# EUROPEAN PATENT APPLICATION

(11) **EP 0 968 648 A1**
(43) Date of publication of application: **05.01.2000**
(21) Application number: 99113609.4
(22) Date of filing: 06.07.1993
(51) Int. Cl.: A01K 67/027, C07K 14/47, C07K 16/42

(54) **Mammalian nonhuman transgenic animal having a constitutive or inducible expression of the human endonexin II gene or its muteins or its fragments**

(30) Priority: 08.07.1992 EP 92401971
(62) Divisional of application: 93915810.1
(71) Applicant: N.V. INNOGENETICS S.A., 9052 Gent (BE)
(72) Inventor: Yap, Sing Hiem, 3221 Nieuwrode (BE)

(57) **Abstract**

The invention relates to a mammalian nonhuman transgenic animal liable to be infected by HBV and moreover liable to suffer from hepatocellular damage, caused by the constitutive or inducible expression of the human endonexin II gene or by the nucleotide sequence coding for its muteins or fragments, the said gene or the said nucleotide sequence being introduced into such a transgenic animal as a model for testing potential drugs.

## Description

The invention relates to polypeptides having hepatitis B virus (HBV) receptor activity.

The invention also relates to new pharmaceutical compositions directed against HBV infection, as well as to new vaccine compositions against HBV. Hepatitis B virus is a virus of worldwide distribution, causing major medical problems such as chronic liver diseases and hepatocellular carcinoma (Ganem, 1982; Schröder et Zentgraf, 1990). Together with duck-, ground squirrel-, and woodchuck hepatitis viruses it belongs to the family of the Hepadnaviridae with which it shares significant hepatotropism and species specificity (Summers et al., 1981; Ganem et Varmus 1987; Leenders et al., 1992).

The HBV envelope consists of three related glycoproteins (surface antigens or HBsAg) differing in size, all of which are the product of a single S gene: (1) a small transmembrane (glyco)protein of 226 AA, termed "major" or small S-protein; (2) the "middle" protein or small S-protein with 55 additional AAs at the N-terminal corresponding to the preS2-region of the S gene; and (3) the "large" (glyco)protein of 389 or 400 AAs consisting of S + preS2 + preS1 regions (108-119 N-terminal AA) (Heerman et al., 1984; Robinson et al., 1987).

The mechanism by which HBV attaches and finally enters the target cell is poorly understood. Many attempts have been made to elucidate the interactions of the HBV envelope proteins with the cell surface membrane. It has been suggested that HBV recognizes specific molecules on the surface of human hepatocytes and that the viral envelope (major, middle and large HBsAg) might play an important role in recognizing these receptor molecules.

Theilmann et Goesser (1991) give an overview of the prior art in which increasing evidence is presented showing that the binding of HBV is mediated by epitopes of the pre-S proteins of the HBV envelope. Indeed, it has been reported that the preS1-region (AA residues 21-47) of large HBsAg contains an attachment site for plasma membranes, derived from HepG2 cells as well as from human liver tissue (Neurath et al., 1986; Pontisso et al., 1989a; Pontisso, et al., 1989b; Neurath et al., 1990; Dash et al., 1992; Petit et al., 1992). In addition, middle HBsAg was found to bind specifically to polymerized human serum albumin (pHSA), within the preS2-region (Machida et al, 1984; Pontisso et al., 1989a; Dash et al., 1991). Furthermore, pHSA binds to hepatocytes, although this binnding is not species specific. A specific binding of major HBsAg to Vero and HepG2 cells has also been demonstrated (Kaiser et al., 1986; Peeples et al., 1987), although this surface protein of HBV was not thought to play a significant role in viral entry into hepatocytes (Pontisso et al., 1989a; Pontisso et al., 1989b).

In a previous study it was observed that major HBsAg was able to bind specifically to intact human hepatocytes (Leenders et al., 1990). In contrast, no specific binding occurs between intact hepatocytes and recombinant large HBsAg, although specific binding between large HBsAg and plasma membrane vesicles was evident (Leenders et al., 1990). Furthermore, individuals vaccinated with major HBsAg are resistant to HBV infection. In addition, a murine monoclonal antibody, RF-HBs-1, directed to a cyclic sequence within major HBsAg (Waters et al., 1986), has been shown to neutralize HBV infection in chimpanzees (Iwarson et al., 1985) and antibodies to this cyclic epitope are always present in sera of vaccinated patients and convalescent patients recovering from HBV infection (Waters et al., 1987). Moreover, anti-major HBsAg monoclonal antibodies have been shown to inhibit the binding of viral particles to HepG2 cells more efficiently than monoclonal antibodies directed to preS1 peptide (Petit et al., 1991). Finally, specific binding of major HBsAg to intact cells has also been found with African green monkey kidney cells (Vero cell line) and human hepatoblastoma HepG2 cell lines (Peeples et al., 1987; Kaiser et al., 1986).

Endonexin II (Annexin V) is a member of a family of structurally related Ca²⁺-dependent phospholipid-binding proteins known as annexins, having molecular weights between 32 to 67 kDa (Klee, 1988; Zaks et Creutz, 1990). The individual proteins in this family were discovered by investigators who had the apparently unrelated goals of identifying mediators of exocytosis, components of the cytoskeleton, inhibitors of phospholipase A2, inhibitors of coagulation and protein-tyrosine kinases. Complete or partial sequence analysis has established that there are at least twelve distinct annexins. These proteins do not have structural similarity with other known Ca²⁺-binding proteins such as the "EF-Hand" family of proteins (Zaks et Creutz, 1990).

Data from a large number of laboratories have also shown that the annexins are found in various tissues of different species (such as human, rat, bovine, chicken,..), e.g. synexin is routinely isolated from liver, but also is present in adrenal medulla, brain, spleen and peripheral blood leucocytes (Klee, 1988). Endonexin II is found in placenta, liver, spleen, lung and intestine (Comera et al., 1989; Walker et al., 1990).

Endonexin II was initially detected as a major intracellular protein that underwent reversible Ca²⁺-dependent binding to placental membranes (Haigler et al., 1987; Schlaepfer et al., 1987; Funakoshi et al., 1987). Independently, the same protein was identified as an in vitro inhibitor of blood coagulation (Funakoshi et al., 1987; Iwasaki et al. 1987; Grundmann et al., 1988) and of phospholipase A2 activity (Pepinsky et al., 1988).

Sequence comparison has shown that the proteins respectively referred to as endonexin II (Kaplan et al., 1988), placental anticoagulant protein or PAP (Funakoshi et al., 1987), PP4 (Grundmann et al., 1988), lipocortin V (Pepinsky et al., 1988) and annexin V (Bianchi et al., 1992) are indeed one and the same protein which will further be referred to as endonexin II.

The clinical use of endonexin II for the treatment of various conditions where an anti-inflammatory effect is desired without the adverse side effects of steroids as well as its use for the diagnosis and treatment of clinical conditions in which it is desirable to interfere with normal phospholipid structure or function (such as in blood coagulation) has been suggested in Patent Application EP-A-0 339 285. Moreover, EP-A-0 330 396 discloses the use of lipocortin V in the treatment of arthritic, allergic, dermatologic, ophthalmic and collagen diseases. The use of vascular anticoagulant protein (VAC=endonexin II), an anticoagulant protein of the annexin family, as an agent for preventing the metastasis of tumors is disclosed in WO 91/07187. In this patent application, a pharmaceutical composition containing VAC as active ingredient, described, preferably ranging from about 0.01 up to about 0.1 mg/kg and more preferably ranging from about 0.01 up to about 0.05 mg/kg bodyweight, administered either intravenously, intramuscularly, or subcutaneously.

The observed in vitro inhibition of blood coagulation and of phospholipase A2 activity seems to be due to sequestration of the phospholipid substrate for these enzymes (Haigler al., 1987; Funakoshi et al., 1987) and may not reflect a physiological function of endonexin II. In Pepinsky et al., 1987, it is stated that in the in vitro phospholipase A2 assays, lipocortin-like proteins are inhibitory; however, the mechanism of inhibition remains controversial. Consequently, the real physiological function of endonexin II remains largely undefined. Furthermore, several observations support the concept of an active role of annexins in modulating the membrane structure (for review see Karshikov et al., 1992). In this respect annexin V has been described to behave like an integral membrane protein (Bianchi et al., 1992). Moreover, it was proven that endonexin II, once bound to the membrane, forms calcium-selective voltage-gated cation channels with prominent selection for Ca²⁺ ions (Rojas et al., 1990).

Endonexin II has extensive sequence homology with the other members of the annexin/lipocortin family, particularly within the consensus sequences that are repeated 4 times within each protein. The major structural difference in these proteins is the variable length of the amino-terminal region (for review see Crompton et al., 1988). The human and rat endonexin II proteins share 92% sequence homology (Kaplan et al., 1988, Pepinsky et al., 1988). In addition, human endonexin II and chicken anchorin CII (Pfaffle et al., 1988), a protein thought to act at the cell surface and mediating adhesion to the extracellular matrix component collagen, also have striking structural similarities (Haigler et al., 1989).

As described above, many investigations have been concerned with the elucidation of a possible receptor for HBV present on human hepatocytes, mainly based on the evidence that the binding of virus is mediated by epitopes of the pre-S proteins of the HBV envelope. However, no direct biochemical evidence as to the existence of such a receptor could be found. In opposition to this theory, some of the above-mentioned data point to the binding of major HBsAg (S-protein) to intact human hepatocytes as well as to Vero and HEPG2 cells.

Consequently, the purpose of the present invention consists in elucidating the nature of a possible HBV receptor present on human hepatocytes, which binds specifically to recombinant and serum-derived large and/or major HBsAg.

The aim of the invention is to provide polypeptides and elements derived therefrom having HBV receptor activity.

Another aim of the invention is to provide pharmaceutical compositions useful in the prevention and treatment of hepatitis B virus infection.

Another aim of the invention is to provide new vaccine compositions against HBV infection.

Another aim of the invention is to provide new means for diagnosing some categories of HBV infection (i.e. individuals expected to develop chronic liver infection).

Another aim of the invention is to provide a screening process enabling the determination of HBV receptor ligands including possible drugs which can be used in the treatment of HBV infection.

The invention relates more particularly to a process for the in vitro determination of the binding between human endonexin II and large and/or major HBsAg comprising the use of:
- a polypeptide having the property of being the receptor of large and/or major HBsAg, and containing or constituted by
   - human endonexin II,
   - or a mutein derived from human endonexin II, with said mutein being such that it is liable to bind to large and/or major HBsAg,
   - or a fragment of endonexin II,
      * with said fragment being such that it is liable to bind to large and/or major HBsAg,
      * or with said fragment being such that it can be recognized by antibodies raised against human endonexin II and capable of neutralizing the HBV infection,
      * or with said fragment being such that it is liable to generate antibodies liable to recognize the above-said endonexin II or large and/or major HBsAg, or a variant polypeptide which corresponds to the above-defined polypeptide, comprising localized mutations such that the polypeptide does not lose its property of being a receptor of large and/or major HBsAg, more particularly with endonexin II peptides DHTLIRVMVSRSEID, DAYELKHALKGAGTNEKVLTEIIASRTPEELRAIKQVYEEEYGSSLE, and DEEKFITIFGTRSVSHLRKVFDKYMTISGFQIEETI,
- or a receptor ligand or antisense peptide with respect to the above-defined endonexin II, or muteins thereof, or fragments thereof,
- or polyclonal or monoclonal antibodies directed against endonexin II, or against the above-defined muteins or against the above-defined fragments,
- or polyclonal or monoclonal antibodies against the above-defined receptor ligands or antisense peptides,
- or anti-idiotype antibodies raised against endonexin II, or against the above-defined muteins, or against the above-defined fragments, or anti-anti-idiotype antibodies raised against endonexin II, or against the above-defined muteins or against the above-defined fragments,
- or anti-idiotype antibodies raised against the above-defined receptor ligand, or antisense peptide, or anti-anti-idiotype antibodies raised against the above-defined receptor ligand or antisense peptides,
- or substances which are active on cells which are transfected by the genes or gene fragments coding for at least one of the polypeptides having a property of being a receptor of large and/or major HBsAg, or coding for at least one of the above-defined muteins or fragments.

The present inventors were able to isolate and characterize the HBV receptor present in human hepatocyte plasma membranes as being endonexin II. Purified infectious HBV particles are only obtainable in small quantities from the blood of infected individuals. The purification procedure for obtaining sufficient amounts of purified infectious HBV particles is difficult without losing the properties of these particles. Therefore, the present invention has preferably made use of the 22 nm, non-infectious plasma derived particles and particles consisting of recombinant, yeast-derived HBsAg which are available in much larger quantities (McAleer et al., 1984). These particles contain only the large and/or major HBsAg envelope protein of HBV.

Human endonexin II is, for instance, disclosed in Kaplan et al. (1988).

In the present text, the expression "human endonexin II" refers to "human liver endonexin II".

The expression "polypeptide having the property of being the receptor of large and/or major HBsAg" corresponds to the fact that the polypeptide can bind to large and/or major HBsAg under the conditions hereabove defined. The nature of the binding is for instance determined by the possibility of DSS (disuccinimidyl suberate) to act as an ideal cross-linker. Consequently, the binding interaction between endonexin II and large and/or major HBsAg is such that the molecules interact with an interaction distance of 11.4 Å.

It is to be noted that the above-mentioned binding conditions are given as an example, and can be modified by the person skilled in the art within limits such that the function of binding remains equivalent to the one defined under the above-mentioned conditions.

The term "mutein derived from human endonexin II" which is liable to bind to major and/or large HBsAg may be experimentally identified under the following conditions:
approximately 15 ng (0.44 pmol) ¹²⁵I-labelled mutein is incubated for 1 h at room temperature with 1 µg large and/or major HBsAg, which has been coated per well of a microtiter plate; specific binding is considered to occur if at least 40%, preferably 50% or more inhibition is obtained with an excess of unlabelled (2 µg) of human endonexin II; binding inhibition is measured by first washing the microtiter plate wells and subsequently counting the amount of residual radioactivity.

The expression "fragment of endonexin II" which is liable to bind to large and/or major HBsAg may be experimentally identified under the following conditions:
approximately 0.44 pmol ¹²⁵I-labelled fragment is incubated for 1 h at room temperature with 1 µg large and/or major HBsAg, which has been coated per well of a microtiter plate; specific binding is considered to occur if at least 40%, preferably 50% or more inhibition is obtained with an excess of unlabelled (2 µg) of human endonexin II; binding inhibition is measured by first washing the microtiter plate wells and subsequently counting the amount of residual radioactivity.

Examples of fragments of endonexin II liable to bind to large and/or major HBsAg are included in the examples section of the present application and include more particularly the peptides designated as F and S having the following amino acid sequences:
Peptide F : DHTLIRVMVSRSEID, (peak F)
Peptide S: DAYELKHALKGAGTNEKVLTEIIASRTPEELRAIKQVYEEEYGSSLE (peak S)
and peptide:DEEKFITIFGTRSVSHLRKVFDKYMTISGFQIEETI (peaks L and N).

The term "receptor ligand" corresponds to a specific ligand, or part of a ligand, having the property of binding to human liver endonexin II; the property of binding to human liver endonexin II can be tested as follows:
radiolabelled large and/or major HBsAg is incubated for 1 h at room temperature with a suspension containing approximately 50,000 human hepatocytes; specific binding is considered to occur if at least 40%, preferably 50% or more inhibition is obtained by adding increased amounts of a receptor ligand; binding inhibition is measeured by first washing the hepatocyte suspendion and subsequently counting the amount of residual radioactivity; as a negative control, cells lacking human endonexin II -for instance rat hepatocytes- are incubated with the radiolabelled receptor ligand as described above.

Preferred illustrations of receptor ligands include for instance antisense peptides as defined below, as well as possible drugs which specifically bind to human liver endonexin II present on hepatocyte plasma membranes.

The term "antisense peptide" is reviewed by Blalock (1990) and by Roubos (1990). In this respect, the molecular recognition theory (Blalock, 1990) states that not only the complementary nucleic acid sequences interact but that, in addition, interacting sites in proteins are composed of complementary amino acid sequences (sense-receptor ligand or sense-antisense peptides). Thus, two peptides derived from complementary nucleic acid sequences in the same reading frame will show a total interchange of their hydrophobic and hydrophilic amino acids when the amino terminus of one is aligned with the carboxy terminus of the other. This inverted hydropathic pattern might allow two such peptides to assume complementary conformations responsible for specific interaction.

The term "receptor ligand" corresponds to any ligand liable to recognize human endonexin II present on human hepatocyte plasma membranes.

The term anti-idiotype antibodies against human endonexin II refers to monoclonal antibodies raised against the antigenic determinants of the variable region of monoclonal antibodies raised against human endonexin II. These antigenic determinants of immunoglobulins are known as idiotypes (sets of iodiotopes) and can therefore be considered to be the "fingerprint" of an antibody (for review see de Préval, 1978; Fleishmann et Davie, 1984). The methods for production of monoclonal anti-idiotypic antibodies have been described by Gheuens et MacFarlin (1982) and are documented in the Examples section of the present application. Monoclonal anti-idiotypic antibodies have the property of forming an immunological complex with the idiotype of the monoclonal antibody against which they were raised. In this respect the monoclonal antibody is referred to as Ab1, and the anti-idiotypic antibody is referred to as Ab2. Ab2 to human endonexin II, or to muteins or fragments derived thereof can thus recognize the receptor binding site on HBV, and can consequently block this HBV binding site, thereby preventing HBV attachment and infection.

The anti-idiotypic antibodies (Ab2) may themselves be used as antigens to produce anti-anti-idiotypic antibodies (Ab3) to these polypeptides which may serve as substitutes for Ab1.

The expression "anti-anti-idiotype antibodies against human endonexin II" refers to antibodies raised against the variable region of anti-idiotype antibodies against human endonexin II.

The invention also relates to a pharmaceutical composition for use in hepatitis infection containing, as active substance, at least one of the following:
- a polypeptide having the property of being the receptor of large and/or major HbsAg, the receptor containing or constituted by
   - a mutein derived from human endonexin II, with said mutein being such that it is liable to bind to large and/or major HBsAg,
   - or a fragment of human endonexin II,
      * with said fragment being such that it is liable to bind to large and/or major HBsAg;
      * or with said fragment being such that it is liable to be recognized by antibodies raised against human endonexin II, capable of neutralizing the HBV infection,
      * or with said fragment being such that it can generate antibodies liable to recognize the above-said endonexin II or a variant polypeptide which corresponds to the above-defined polypeptide, comprising localized mutations such that the polypeptide does not lose its property of being a receptor of large and/or major HBsAg, more particularly with endonexin peptides DHTLIRVMVSRSEID, DAYELKHALKGAGTNEKVLTEIIASRTPEELRAIKQVYEEEYGSSLE, and DEEKFITIFGTRSVSHLRKVFDKYMTISGFQIEETI,
- or a ligand or antisense peptide with respect to the receptor defined above, with said ligand being defined above
- or polyclonal or monoclonal antibodies, preferably human monoclonal antibodies, against the ligands or antisense peptides as defined above,
- or anti-idiotype antibodies raised against human endonexin II, or against the muteins as defined above, or against the fragments as defined above, or anti-anti-idiotype antibodies raised against human endonexin II, or against the muteins as defined above, or against the fragments as defined above,
- or anti-idiotype antibodies raised against the above-defined receptor ligand or antisense peptide or anti-anti-idiotype antibodies raised against the ligand as defined above or antisense peptide as defined above,
- or substances which are active on cells which are transfected by the genes or gene fragments coding for one at least of the polypeptides having a property of being a receptor of large and/or major HBsAg, or coding for at least one of the muteins as defined above or fragments as defined above.

The expression "humanized versions of mouse monoclonal antibodies" corresponds to monoclonal antibodies made by means of recombinant DNA technology, departing form parts of mouse and/or human genomic DNA sequences coding for H and L chains or from cDNA clones coding for H and L chains.

The expression "human monoclonal antibodies against receptor ligands or antisense peptides" relates to monoclonal antibodies prepared for instance by means of human peripheral blood lymphocytes (PBL) repopulation of severe combined immune deficiency (SCID) mice (for recent review see Duchosal et al. 1992). In short, SCID-mice are repopulated with a human hematopoietic system (capable of generating all human B cells). After repopulation, these SCID-mice are for instance immunized with HBV, inducing human monoclonal antibody formation. Alternatively, human monoclonal antibodies can be prepared by taking B-cells from a convalescent patient recovering from HBV infection, likely to contain B-cells that have reacted to HBV, and preferably carrying or secreting Ig with neutralising capacity, and immortalizing these B-cells with the use of Eppstein-Barr virus (EBV) or retrovirus transfection. Alternatively, the same B-cells or a pool of white blood cells from different persons, not necessarily infected with HBV, is taken and a cDNA library (either total, but preferentially by PCR so that only H chain cDNA and C chain cDNA is formed) is prepared from the isolated mRNA. Finally the CDNA_{H}-phages and the cDNA_{L}-phages are combined and screening for recombinant IgG anti-HBV can be performed.

Human monoclonal antibodies against human endonexin II, or muteins thereof, or fragments thereof can be obtained by first immunizing a patient with a fragment or mutein of human endonexin II -liable to induce an immune response in the patient-, taking B-cells from this patient and repopulating SCID-mice with these B-cells. Upon immunization of these repopulated SCID-mice with the same endonexin II fragement or mutein, large amounts of endonexin II human monoclonal antibodies can be obtained as defined above.

In the muteins of endonexin II in the present invention, the following amino acids can be replaced as shown in Table 1.

**TABLE 1**

| Amino acids | Synonymous groups |
|---|---|
| Ser | Ser, Thr, Gly, Asn |
| Arg | Arg, His, Lys, Glu, Gln |
| Leu | Leu, Ile, Met, Phe, Val, Tyr |
| Pro | Pro, Ala, Thr, Gly |
| Thr | Thr, Pro, Ser, Ala, Gly, His, Gln |
| Ala | Ala, Pro, Gly, Thr |
| Val | Val, Met, Ile, Tyr, Phe, Leu, Val |
| Gly | Gly, Ala, Thr, Pro, Ser |
| Ile | Ile, Met, Leu, Phe, Val, Ile, Tyr |
| Phe | Phe, Met, Tyr, Ile, Leu, Trp, Val |
| Tyr | Tyr, Phe, Trp, Met, Ile, Val, Leu |
| Cys | Cys, Ser, Thr |
| His | His, Gln, Arg, Lys, Glu, Thr |
| Gln | Gln, Glu, His, Lys, Asn, Thr, Arg |
| Asn | Asn, Asp, Ser, Gln |
| Lys | Lys, Arg, Glu, Gln, His |
| Asp | Asp, Asn, Glu |
| Glu | Glu, Gln, Asp, Lys, Asn, His, Arg |
| Met | Met, Ile, Leu, Phe, Val |

The substances according to this preferred embodiment of the invention compete with human endonexin II present on hepatocyte plasma membrane for the endonexin II-binding site on the HBV. These polypeptides, muteins and fragments are pharmacologically active insofar as they block the virus, and thus prevent the HBV from attaching to and infecting the hepatocytes.

The receptor ligand or antisense peptides of the invention have the properties of competing with the virus for the HBV binding site present on the molecule(s) of the endonexin II HBV receptor. These receptor ligand or antisense peptides are pharmacologically active insofar as they block the HBV binding site of human endonexin II present on human hepatocytes, and thus protect the hepatocyte from virus attachment and infection.

Anti-idiotype antibodies raised against endonexin II or against its muteins or against the fragments as defined above recognize the receptor binding site on HBV and can consequently block this binding site of HBV, thereby preventing HBV attachment and infection.

Anti-anti-idiotype antibodies raised against human endonexin II or against its muteins or against the fragments as defined above are competitors with the virus and are pharmacologically active insofar as they block the HBsAg binding site of human endonexin II of hepatocytes, and protect the hepatocytes from the virus.

The expression "the HBV binding site" more specifically relates to the HBsAg binding site.

Anti-idiotype antibodies raised against receptor ligand or antisense peptides are competitors with the virus and are pharmacologically active insofar as they block the HBsAg binding site of human endonexin II of hepatocytes and protect the hepatocytes from the virus.

Polyclonals or monoclonals antibodies as well as anti-anti-idiotype antibodies raised against the receptor ligand or antisense peptides compete with human endonexin II on hepatocytes and are pharmacologically active insofar as they block the endonexin II attachment site of the virus and prevent it from attacking the hepatocytes.

Humanized versions of mouse monoclonal antibodies as well as human monoclonal antibodies raised against endonexin II, or muteins thereof, or fragments thereof, compete with HBV for the HBV binding site of the HBV receptor, endonexin II, and can be pharmacologically efficient for treatment or prevention of diseases and/or infections caused by HBV insofar as they block the HBV binding site of endonexin II, and thus protect hepatocytes from HBV attachment and infection. This type of pharmaceutical composition can be very useful for chronic HBV carriers who have to undergo liver transplantation.

The pharmaceutical composition of the invention can be preferably used for treatment of chronic HBV carriers or carriers who are chronically and/or acutely infected with HBV.

According to a preferred embodiment, the amount of the active substance in the pharmaceutical compositions of the invention is equivalent to a dosage of endonexin II from about 0.6 to about 50 mg/kg bodyweight, and preferably from about 1 to about 30 mg/kg bodyweight, and more preferably from 10 to 15 mg/kg bodyweight.

The mode of administration of these pharmaceutical compositions is enteral or preferably parenteral, i.e., intravenous, intraperitoneal, intramuscular, or subcutaneous, with the intravenous administration being preferred.

The active substances of these pharmaceutical compositions may be administered alone, without a carrier vehicle; however, they may also be administered with pharmaceutically acceptable non-toxic carriers or diluents, the proportions of which are determined by the suitability and chemical nature of the particular carrier.

The invention also relates to vaccine composition containing, as active substance, muteins or fragments of endonexin as defined above, more particularly endonexin peptides
DHTLIRVMVSRSEID,
DAYELKHALKGAGTNEKVLTEIIASRTPEELRAIKQVYEEEYGSSLE, and
DEEKFITIFGTRSVSHLRKVFDKYMTISGFQIEETI.

The invention also relates to vaccine compositions containing, as active substance,
* receptor ligands or antisense peptides as defined above,
* or anti-idiotype antibodies raised against the receptor ligand or antisense peptides as defined above.

The HBV vaccine compositions according to the present invention which contain either muteins or fragments of endonexin as defined above or receptor ligands or antisense peptides or anti-idiotypic antibodies against the receptor ligands and antisense peptides have certain advantages over the already known HBV vaccine compositions, because they are more likely to give rise to an immune response in those HBV infected patients who would not respond to the already existing HBV vaccine compositions.

Vaccine compositions of the prior art are for instance, described in Hilleman *et al.* (1983) (particles prepared from the serum of chronically infected HBV carriers), in McAleer *et al.* (1984) (recombinant yeast vaccine) and are reviewed in Theilmann et Goesser (1991).

In the vaccine compositions of the invention, the active substance is present in an amount of endonexin II binding sites equivalent to 10-100 µg HBsAg or approximately 0.1 nmole to 1 µmole active substance per kg of bodyweight per immunization, preferably 0.1 nmole to 100 nmole, most preferably 1 nmole to 100 nmole.

According to an advantageous embodiment of the invention, the antibodies are preferably in an amount of 10 µg to 1 mg/kg of body weight.

The polypeptides of the invention can be prepared by the culture of a cellular host in an appropriate medium which has previously been transformed by a recombinant vector coding for the polypeptides of the invention and by recovering the polypeptide produced by said transformed cellular host, from the above-said culture.

The polypeptides and more particularly the peptides of the invention can also be prepared by classical chemical synthesis.

The synthesis can be carried out in homogeneous solution or in solid phase.

For instance, the synthesis technique in homogeneous solution which can be used is the one described by Houbenweyl in the book entitled "Methode der organischen chemie" (Method of organic chemistry) edited by E. Wunsh, vol. 15-I et II. THIEME, Stuttgart 1974.

The polypeptides of the invention can also be prepared in solid phase according to the methods described by Atherton and Shepard in their book entitled "Solid phase peptide synthesis" (IRL Press, Oxford, 1989).

The antisense peptides can be prepared as described in Ghiso et al. (1990). By means of this technology it is possible to logically construct a peptide having a physiological relevant interaction with a known peptide by simple nucleotide sequence analysis for complementarity, and synthesize the peptide complementary to the binding site.

The monoclonal antibodies of the invention can be produced by any hybridoma liable to be formed according to classical methods from splenic cells of an animal, particularly of a mouse or rat, immunized against the endonexin II or muteins thereof or fragments thereof or receptor ligand or antisense peptides defined above on the one hand, and of cells of a myeloma cell line on the other hand, and to be selected by the ability of the hybridoma to produce the monoclonal antibodies recognizing the polypeptides which has been initially used for the immunization of the animals.

The antibodies involved in the invention can be labelled by an appropriate label of the enzymatic, fluorescent or radioactive type.

The anti-idiotype antibodies raised against human endonexin II, or against its muteins, or against the fragments as defined above, or anti-anti-idiotype antibodies raised against human endonexin II, or against its muteins, or against the fragments as defined above, or the humanized versions of mouse monoclonal antibodies or human monoclonal antibodies against human endonexin II or against its muteins or against the fragments as defined above can be prepared as mentioned above.

The anti-idiotype antibodies raised against the receptor ligand or antisense peptides as defined above, or anti-anti-idiotype antibodies raised against the receptor ligand or antisense peptides as defined above or human monoclonal antibodies raised against the receptor ligands or antisense peptides can be prepared as mentioned above.

In order to carry out the expression of the polypeptides of the invention in a bacteria, such as E. coli or in an eukaryotic cell such as S. cerevisiae, or in cultured vertebrate or invertebrate hosts such as insect cells, Chinese Hamster Ovary (CHO), COS1, BHK, and MDCK cells, the following steps are carried out:
- transformation of an appropriate cellular host with a vector, particularly a plasmid, a cosmid, a phage or a virus (vaccinia A or B), in which a nucleotide sequence coding for one of the polypeptides of the invention has been inserted (insert) under the control of the appropriate regulatory elements, particularly a promoter recognized by the polymerases of the cellular host and, in the case of a prokaryotic host, an appropriate ribosome binding site (RBS), enabling the expression in said cellular host of said nucleotide sequence,
- culture of said transformed cellular host in conditions enabling the expression of said insert.

For the expression in yeast, the regulatory elements can comprise, the promoters of 3-phosphoglycerate kinase or other glycolytic enzymes, suitable polyadenylation and termination signals, as well as a suitable origin of replication. Expression vectors for cultured cells include an origin of replication, a promoter located in front of the gene to be expressed, a translation start site, RNA splice sites, a polyadenylation site, and transcriptional termination signals.

For the expression in E. coli, the regulatory elements should comprise all the necessary signals for transcription and translation. They can comprise a promotor, either synthetic or derived from a natural source, which is functional in E. coli. Examples of widely used promotors for expression in E. coli are the lac, the trp, the tac and the lambda Pl and Pr promoters. They can also comprise a ribosome binding site, either synthetic or from a natural source, allowing translation and hence expression of a downstream coding sequence.

The invention also relates to a process for detecting the capacity of a molecule to behave as a receptor ligand with respect to any one of the receptor polypeptides as defined above or to any of the endonexin II peptides as mentioned above and characterized by:
- contacting the molecule with a cellular host which has previously been transformed by a vector itself modified by an insert coding for one of the above-said polypeptides, the host bearing at its surface one or more sites specific for this polypeptide, if need be after contacting the expression of said insert, with said contacting being carried out under conditions enabling the formation of a binding between one at least of the above-said specific sites and said molecule, if this latter presents an affinity with respect to said polypeptide,
- detecting the possibly formed complex of the ligand-polypeptide type or of the endonexin II peptides as mentioned above.

It is to be understood that in the present text the expression "ligand" means "receptor ligand".

In this respect, a screening process can be developed for new anti-hepatitis drugs.

The invention also relates to a process for detecting the affinity of the polypeptides as defined or of the endonexin II peptides as mentioned above for one or several receptor ligands, characterized by:
- transforming an appropriate host cell with a vector, particularly a plasmid or a phage, in which a nucleotide sequence coding for one of the above-defined polypeptides or peptides has been previously inserted, (insert) under the control of regulatory elements, particularly a promoter recognized by the polymerase of the host cell and enabling the expression of said nucleotide sequence in said host cell,
- culturing the transformed host cell under conditions enabling the expression of said insert, and the transportation of said polypeptide which has been expressed towards the membrane so that the sequences needed for interaction with the ligand be exposed to the surface of the transformed host cell,
- contacting this host cell with determined ligands,
- detecting an affinity reaction between said transformed host cell and said determined ligands.

The above-described process also enables identification of the fragments defined above and which contain only part of the sequence of endonexin II.

This identification consists in using inserts smaller than the nucleic acid coding for endonexin II in carrying out the steps relative to the expression, transport of the expression product, and exposure of the expression product to the membrane of the transformed host cell. When expression, transport of the expression product and its exposure to the membrane, as well as the reaction with ligands such as previously defined are obtained, it is possible to determine said fragments. Consequently, the absence of reaction with the ligands, such as previously defined, by using fragments of smaller size than the size of the complete sequence, would show that some essential sites have been eliminated.

The invention also relates to a kit for the detection of the possible affinity of a ligand with respect to any one of the polypeptides of the invention, with said kit containing:
- a culture of cellular hosts transformed by a modified vector such as defined above or the preparation of membranes of said cellular hosts,
- physical or chemical means to induce the expression of the nucleotidic sequence contained in the modified vector when the promoter placed upstream from this sequence is a promoter inducible by said physical or chemical means, and to obtain a protein,
- one or several control ligands having determined affinities for the above-said polypeptide,
- physical or chemical means for characterizing the biological activity of the expressed protein.

The invention also relates to a process for screening drugs liable to be used in the treatment of HBV infection.

The invention also relates to a process for the in vitro detection of large and/or major HBsAg comprising:
- contacting a biological sample liable to contain HBV with a fixed amount of (i) human endonexin II, or of muteins thereof, or of a fragment thereof as defined above and more particularly endonexin peptides:
   DHTLIRVMVSRSEID,
   DAYELKHALKGAGTNEKVLTEIIASRTPEELRAIKQVYEEEYGSSLE, and
   DEEKFITIFGTRSVSHLRKVFDKYMTISGFQIEETI and a fixed amount of (ii) one of the receptor ligand, or antisense peptide as defined above and acting as a competitor of HBsAg, under conditions enabling the formation of a complex between (i) human endonexin II or its functional counterpart as defined above and (ii) receptor ligand or antisense peptide and between human endonexin II and large and/or major HBsAg, and with either component (i) or (ii) carrying a detectable label and either component being possibly immobilized,
- detecting the amount of complex possibly formed between (i) human endonexin II or its functional counterpart and (ii) the receptor ligand, or antisense peptides and the complex formed between (i) human endonexin II or its functional counterpart and (ii) large and/or major HBsAg,
- deducing the amount of HBV which is present in said sample as the decrease of complex formation with respect to a control sample known to be free from HBV.

The biological sample which can be used includes all type of body fluids or biopsy materials, preferably blood samples, more preferably serum and/or plasma samples.

The invention also relates to a process for the in vitro determination of hepatocellular damage in a biological sample taken from a body tissue (biopsy), plasma or serum comprising:
- contacting a biological sample liable to contain human endonexin II from damaged hepatocytes,
   - either with a plate coated with receptor ligand or antisense peptides such as defined above,
   - or with a plate coated with polyclonal and/or monoclonal antibodies, preferably human monoclonals or humanized versions of mouse monoclonals, directed against human endonexin II, or against the muteins of human endonexin II as defined above, or against the fragments derived from human endonexin II as defined above,
   - or with a plate coated with anti-anti-idiotype antibodies raised against human endonexin II, or against the muteins of human endonexin II as defined above, or against the fragments derived from human endonexin II as defined above,
   - or with a plate coated with anti-idiotype antibodies raised against the receptor ligand, or antisense peptides as defined above,
- detecting the complex formed either between human endonexin II and the receptor ligand or antisense peptides, or between human endonexin II and the monoclonal or polyclonal antibodies, or between human endonexin II and the above-defined anti-anti-idiotype antibodies or between human endonexin II and the above-defined anti-idiotype antibodies.

In this respect, hepatocellular damage is known to occur by toxic immunologic or pathogenic mechanisms and in particular in the event of chronic liver infection caused by HBV, and more specifically in the event of liver cirrhosis and hepatocellular carcinoma. Consequently, the in vitro determination techniques can be used to diagnose and monitor the disease state.

The invention also relates to a diagnostic kit for the in vitro determination of HBV containing:
- at least one microplate for deposition thereon of a product comprising:
   * human endonexin II or muteins thereof such as defined above, or fragments derived from human endonexin II such as defined above, and more particularly endonexin peptides:
      DHTLIRVMVSRSEID,
      DAYELKHALKGAGTNEKVLTEIIASRTPEELRAIKQVYEEEYGSSLE, and
      DEEKFITIFGTRSVSHLRKVFDKYMTISGFQIEETI,
   * or anti-idiotype antibodies raised against human endonexin II, or against muteins thereof such as defined above, or against fragments derived from human endonexin II such as defined above,
- a preparation containing the sample to be diagnosed and liable to contain HBsAg, together with a competitor to HBsAg, such as:
   * receptor ligand or antisense peptide as defined above or,
   * monoclonal or polyclonal antibodies, preferably human monoclonals or humanized versions of mouse monoclonals, against human endonexin II, or against the muteins of endonexin II as defined above, or against the fragments derived from human endonexin II as defined above, and more particularly endonexin peptides:
      DHTLIRVMVSRSEID,
      DAYELKHALKGAGTNEKVLTEIIASRTPEELRAIKQVYEEEYGSSLE, and
      DEEKFITIFGTRSVSHLRKVFDKYMTISGFQIEETI,
   * or anti-anti-idiotype antibodies raised against human endonexin II,
   * or anti-idiotype antibodies raised against the receptor ligand or antisense peptides as defined above,
- appropriate buffer solutions for carrying out the reaction between HBsAg and the product deposited on the plate,
- appropriate markers for the determination of the complex formed between HBsAg and the product deposited on the plate.

According to an alternative embodiment the microplate may also contain deposited theron :
* or polyclonal or monoclonal antibodies, preferably human monoclonals, directed against the receptor ligand or antisense peptides as defined above,
* or anti-anti-idiotype antibodies raised against the receptor ligand, or antisense peptides as defined above.

The invention also relates to a diagnostic kit for the in vitro determination of endonexin II containing:
- at least one microplate for deposition thereon of a product comprising:
   * receptor ligand or antisense peptides such as defined above or,
   * polyclonal or monoclonal antibodies, preferably human monoclonals or humanized versions of mouse monoclonals, directed against human endonexin II, or against the muteins of human human endonexin II as defined above, or against the fragments derived from human endonexin II as defined above and more particularly endonexin peptides:
      DHTLIRVMVSRSEID,
      DAYELKHALKGAGTNEKVLTEIIASRTPEELRAIKQVYEEEYGSSLE, and
      DEEKFITIFGTRSVSHLRKVFDKYMTISGFQIEETI,
   * anti-anti-idiotype antibodies raised against human endonexin II, or against the muteins of human endonexin II as defined above, or against the fragments derived from human endonexin II as defined above and more particularly endonexin peptides
      DHTLIRVMVSRSEID,
      DAYELKHALKGAGTNEKVLTEIIASRTPEELRAIKQVYEEEYGSSLE, and
      DEEKFITIFGTRSVSHLRKVFDKYMTISGFQIEETI,
- a preparation containing the sample to be diagnosed, possibly together with a competitor of human endonexin II, such as:
   - polyclonal or monoclonal antibodies raised against the receptor ligand or antisense peptides as defined above,
   - or anti-idiotype antibodies raised against human endonexin II, or against the muteins of human endonexin II, or against the fragments derived from human endonexin II as defined above and more particularly endonexin peptides: DHTLIRVMVSRSEID, DAYELKHALKGAGTNEKVLTEIIASRTPEELRAIKQVYEEEYGSSLE, and DEEKFITIFGTRSVSHLRKVFDKYMTISGFQIEETI or,
   - anti-idiotype antibodies raised against the receptor ligand or antisense peptides as defined above,
- a preparation containing the sample to be diagnosed, possibly together with a competitor of human endonexin II which is sought, such as:
   - polyclonal or monoclonal antibodies, preferably human monoclonal antibodies, raised against the receptor ligand or antisense peptides as defined above,
   - or anti-idiotype antibodies raised against human endonexin II, or against the muteins of human endonexin II as defined above, or against the fragments derived from human endonexin II as defined above,
   - or anti-anti-idiotype antibodies raised against the receptor ligand or antisense peptides as defined above,
- appropriate buffer solution for carrying out the immunological reactions between human endonexin II and the product deposited on the plate,
- appropriate marker to detect the formation of a complex between human endonexin II and the product deposited on the plate.

This type of kit would be suited for diagnosing and monitoring the disease state of chronic liver infections.

The invention also relates to the use of:
- a polypeptide as defined above,
- or of a receptor ligand or antisense peptide as defined above,
- or of polyclonal or monoclonal antibodies, preferably human monoclonal antibodies or humanized versions of mouse monoclonal antibodies, raised against human endonexin II as defined above, or against muteins thereof as defined above, or against fragments thereof as defined above and more particularly endonexin peptides: DHTLIRVMVSRSEID, DAYELKHALKGAGTNEKVLTEIIASRTPEELRAIKQVYEEEYGSSLE, and DEEKFITIFGTRSVSHLRKVFDKYMTISGFQIEETI,
- or of anti-idiotype antibodies against human endonexin II as defined above, or against muteins thereof as defined above, or against fragments thereof as defined above and more particularly endonexin peptides:
   DHTLIRVMVSRSEID,
   DAYELKHALKGAGTNEKVLTEIIASRTPEELRAIKQVYEEEYGSSLE, and
   DEEKFITIFGTRSVSHLRKVFDKYMTISGFQIEETI,
- or anti-anti-idiotype antibodies against human endonexin II, as defined above, or against muteins thereof as defined above, or against fragments thereof as defined above and more particularly endonexin peptides:
   DHTLIRVMVSRSEID,
   DAYELKHALKGAGTNEKVLTEIIASRTPEELRAIKQVYEEEYGSSLE, and
   DEEKFITIFGTRSVSHLRKVFDKYMTISGFQIEETI,
- or anti-idiotype antibodies raised against the anti-sense peptides as defined above,
- or anti-anti-idiotype antibodies against the receptor ligand or antisense peptides as defined above,
- or the substances as defined above,
   for the preparation of a drug useful for the treatment of hepatitis B.

The invention also relates the use of:
- a polypeptide as defined above,
- or of a receptor ligand or antisense peptide as defined above,
- or of polyclonal or monoclonal antibodies, preferably human monoclonal antibodies or humanized versions of mouse monoclonal antibodies, raised against human endonexin II as defined above, or against muteins thereof as defined above, or against fragments thereof as defined above and more particularly endonexin peptides DHTLIRVMVSRSEID, DAYELKHALKGAGTNEKVLTEIIASRTPEELRAIKQVYEEEYGSSLE, and DEEKFITIFGTRSVSHLRKVFDKYMTISGFQIEETI,
- or of anti-idiotype antibodies against human endonexin II as defined above, or against muteins thereof as defined above, or against fragments thereof as defined above and more particularly endonexin peptides:
   DHTLIRVMVSRSEID,
   DAYELKHALKGAGTNEKVLTEIIASRTPEELRAIKQVYEEEYGSSLE, and
   DEEKFITIFGTRSVSHLRKVFDKYMTISGFQIEETI,
- or anti-anti-idiotype antibodies against human endonexin II, as defined above, or against muteins thereof as defined above, or against fragments thereof as defined above and more particularly endonexin peptides:
   DHTLIRVMVSRSEID,
   DAYELKHALKGAGTNEKVLTEIIASRTPEELRAIKQVYEEEYGSSLE, and
   DEEKFITIFGTRSVSHLRKVFDKYMTISGFQIEETI,
- or anti-idiotype antibodies raised against the anti-sense peptides as defined above,
- or anti-anti-idiotype antibodies against the receptor ligand or antisense peptides as defined above,
- or the substances as defined above,
   for the preparation of a vaccine against hepatitis B infection.

The invention also relates to the use of:
- a polypeptide as defined above,
- or of a receptor ligand or antisense peptide as defined above,
- or of polyclonal or monoclonal antibodies against human endonexin II as defined above, or against muteins thereof as defined above, or against fragments thereof as defined above and more particularly endonexin peptides:
   DHTLIRVMVSRSEID,
   DAYELKHALKGAGTNEKVLTEIIASRTPEELRAIKQVYEEEYGSSLE, and
   DEEKFITIFGTRSVSHLRKVFDKYMTISGFQIEETI,
- or of anti-idiotype antibodies against human endonexin II as defined above, or against muteins thereof as defined above, or against fragments thereof as defined above and more particularly endonexin peptides:
   DHTLIRVMVSRSEID,
   DAYELKHALKGAGTNEKVLTEIIASRTPEELRAIKQVYEEEYGSSLE, and
   DEEKFITIFGTRSVSHLRKVFDKYMTISGFQIEETI,
- or anti-anti-idiotype antibodies against human endonexin II, as defined above, or against muteins thereof as defined above, or against fragments thereof as defined above and more particularly endonexin peptides:
   DHTLIRVMVSRSEID,
   DAYELKHALKGAGTNEKVLTEIIASRTPEELRAIKQVYEEEYGSSLE, and
   DEEKFITIFGTRSVSHLRKVFDKYMTISGFQIEETI,
- or anti-idiotype antibodies raised against the anti-sense peptides as defined above,
- or anti-anti-idiotype antibodies against the receptor ligand or antisense peptides as defined above,
- or the substances as defined above,
   for the preparation of diagnostic means of hepatitis B virus infection.

The invention also relates to a mammalian nonhuman transgenic animal liable to be infected by HBV and moreover liable to suffer from hepatocellular damage, caused by the constitutive or inducible expression of the human endonexin II gene or by the nucleotide sequences coding for its muteins or fragments and more particularly endonexin peptides:
DHTLIRVMVSRSEID,
DAYELKHALKGAGTNEKVLTEIIASRTPEELRAIKQVYEEEYGSSLE, and
DEEKFITIFGTRSVSHLRKVFDKYMTISGFQIEETI,
the said gene or the said nucleotide sequence being introduced into such a transgenic animal as a model for testing potential drugs which are able to modulate the expression of HBV and/or the binding of HBV to human endonexin II, to its muteins, or to its fragments as defined above and more particularly endonexin peptides DHTLIRVMVSRSEID, DAYELKHALKGAGTNEKVLTEIIASRTPEELRAIKQVYEEEYGSSLE, and DEEKFITIFGTRSVSHLRKVFDKYMTISGFQIEETI.

A transgenic nonhuman mammalian animal can be prepared according to the protocol described in International Review of Cytology, Gordon JW, vol. 115, p.171-222 (1989).

### BRIEF DESCRIPTION OF THE FIGURES

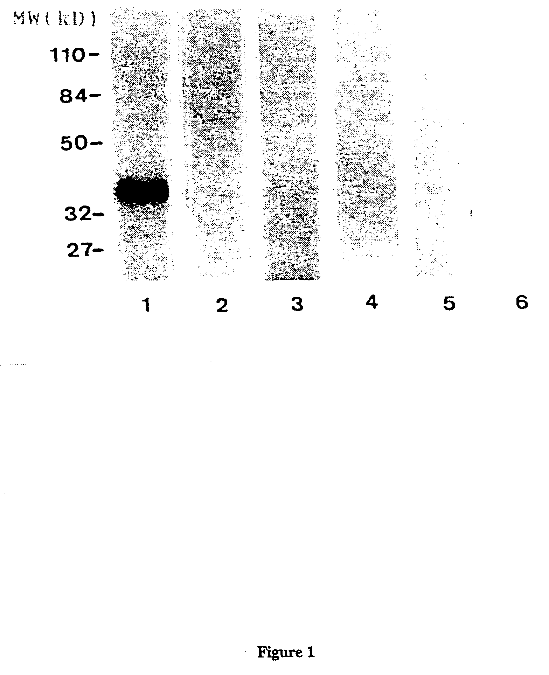

Figure 1 Autoradiogram of an SDS-PAGE analysis of proteins bound to recombinant major HBsAg coated on a microtiter plate (Lanes 1-4) and to BSA coated on the same plate (Lanes 5-6).
   Lanes 1-2: Binding of ¹²⁵I-labelled human plasma membrane proteins in the absence (Lane 1) and the presence (Lane 2) of excess of unlabelled plasma membrane proteins.
   Lanes 3-4: Binding of ¹²⁵I-labelled rat plasma membrane proteins, in the absence (Lane 3) and the presence (Lane 4) of excess of unlabelled plasma membrane proteins.
   Lanes 5-6: Binding of ¹²⁵I-labelled human plasma membrane proteins to BSA-coated wells in the absence (Lane 5) and the presence (Lane 6) of excess of unlabelled plasma membrane proteins.

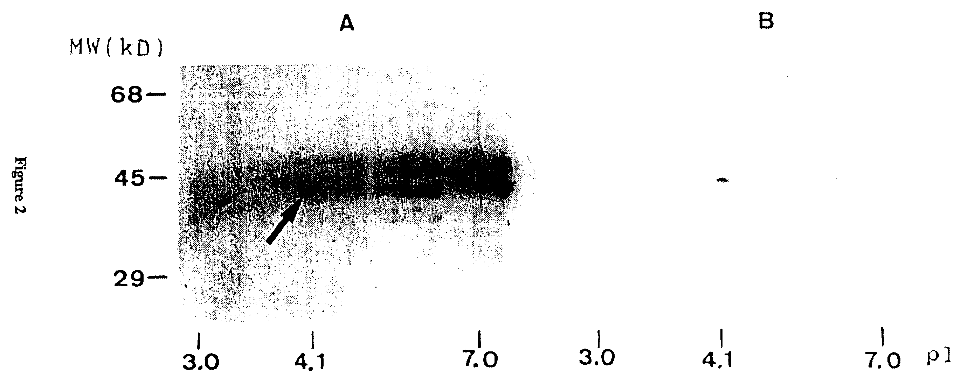

Figure 2A Two-dimensional gel electrophoresis of a 34 kD protein fraction, isolated by preparative PAGE of human liver plasma membrane proteins. The relative acidic protein, with pI value of 4.1 (arrow) has been identified as the major HBsAg binding-protein. (see also fig. 2B)
Figure 2B Autoradiogram of IEF/SDS-PAGE analysis of ¹²⁵I-labeled human liver plasma membrane protein after binding to major HBsAg immobilized on high bond ELISA dishes, washing and elution. The single spot on the figure corresponds to a protein with MW of 34 kD and pI value of 4.1. (see also fig. 2A).

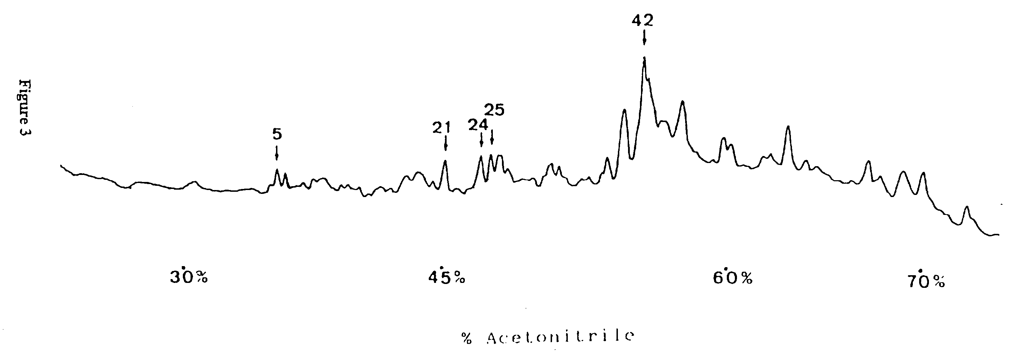

Figure 3 Elution pattern of reversed phase HPLC separated peptides after limited acid hydrolysis of 34 kD protein in gel matrix. This 34 kD protein has been obtained from two dimensional gel electrophoresis as indicated in figure 2. Peak fractions indicated by arrows were analysed for amino acid sequences and used for databank search.

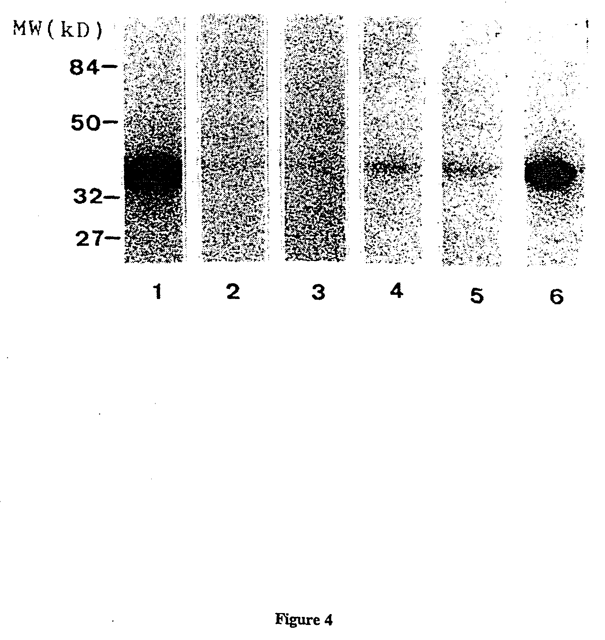

Figure 4 Autoradiogram of SDS-PAGE analysis of human liver E-II bound to recombinant major HBsAg, immobilized on high bond ELISA dishes in the absence or presence of competitors. ¹²⁵I-labeled human liver E-II was used in the absence (Lane 1) or in the presence (Lane 2) of excess unlabeled E-II and, in other experiments, in the presence of excess recombinant major HBsAg (Lane 3), or excess unlabeled human liver plasma membrane proteins (Lane 4). Pre-incubation of labeled E-II with rabbit anti-E-II IgG (1:200) inhibits binding of E-II to major HBsAg (Lane 5). No inhibition was found when pre-incubation was performed with pre-immune IgG (Lane 6). E-II represents endonexin-II.

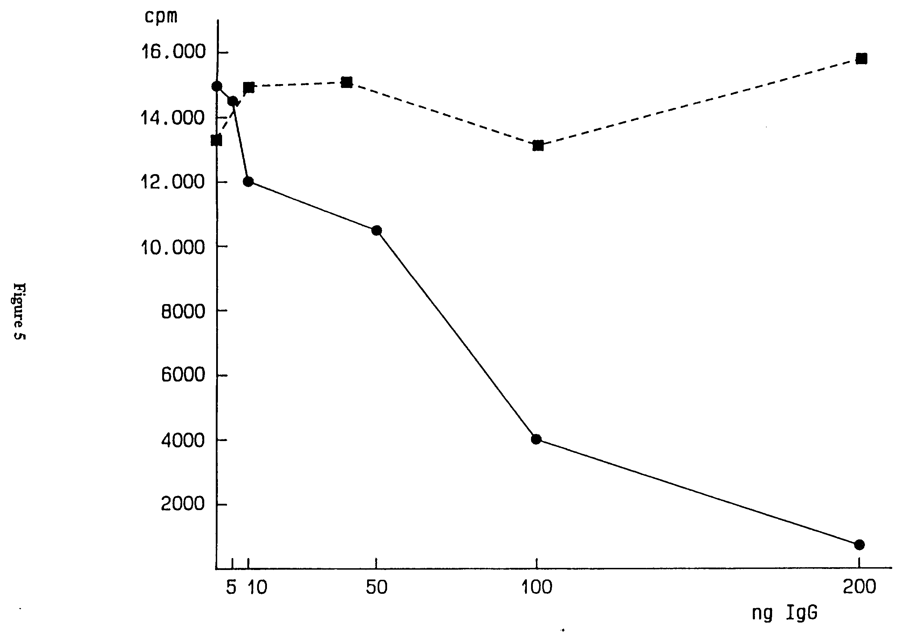

Figure 5 Binding of radiolabelled E-II (cpm) to major HBsAg in the presence of varying concentrations of rabbit anti-E-II IgG (●-●) or comparable concentrations of rabbit pre-immune IgG (■--■).

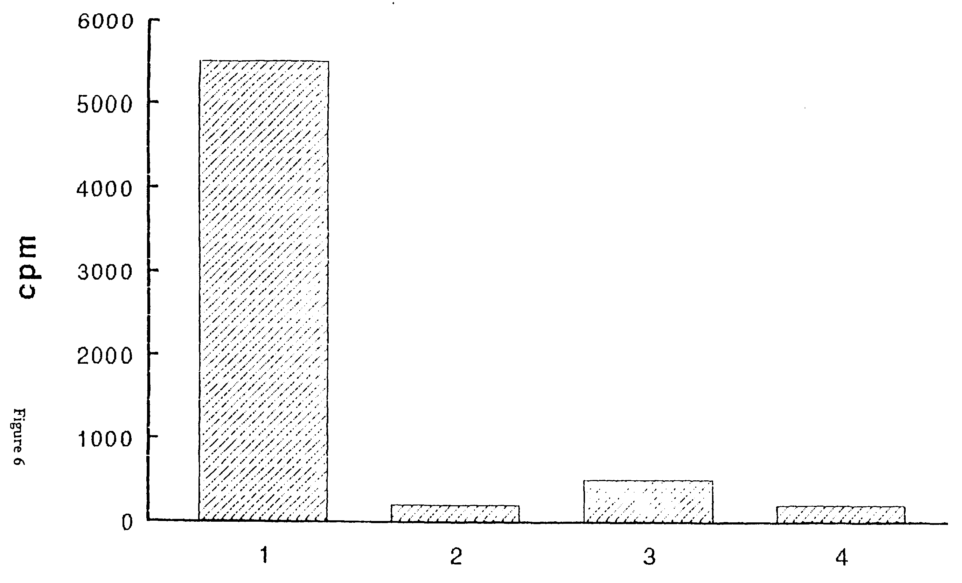

Figure 6 Binding of radiolabelled endonexin II (cpm) to major HBsAg and to BSA, immobilized on solid phase. Lane 1 and 2 represent the binding of 10 ng radiolabelled human liver endonexin II to major HBsAg and BSA respectively. Lane 3 and 4 represent results of the same experiment with 10 ng radiolabelled rat liver endonexin II.

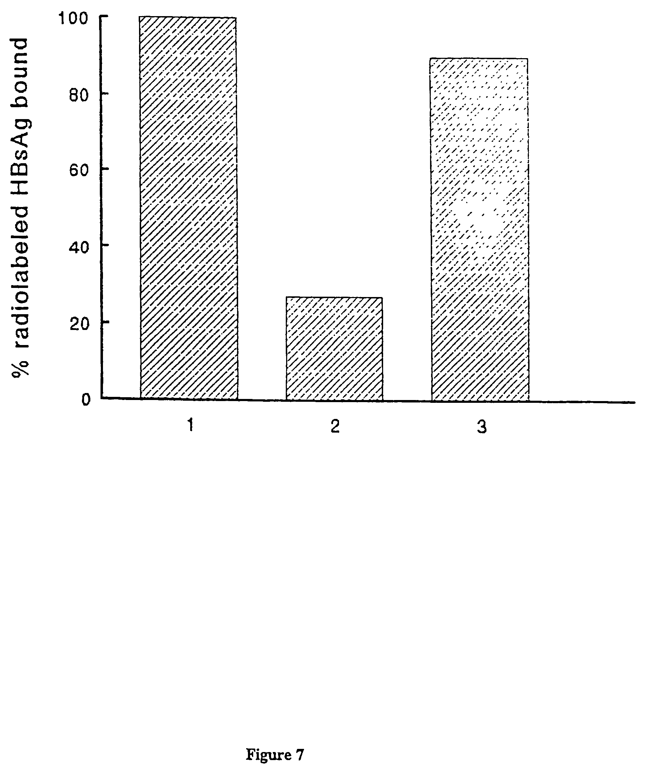

Figure 7 Specific binding of radiolabelled major HBsAg to intact cultured human hepatocytes in the presence and the absence of competitors. Lane 1 represents specific binding of radiolabelled HBsAg in the absence of competitor (control). Lane 2 represents binding of radiolabelled major HBsAg in the presence of endonexin II. Lane 3 represents binding of radiolabelled HBsAg in the presence of control protein (mouse IgG).

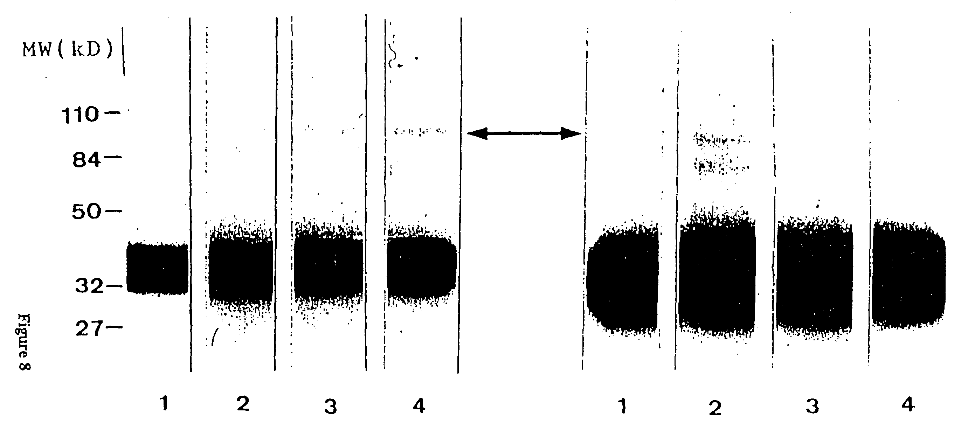

Figure 8 *Left panel.* Autoradiogram of SDS-PAGE analysis of cross-linking experiments using ¹²⁵I-labelled endonexin II and recombinant or serum-derived major HBsAg immobilized on solid phase. Crosslinking of endonexin II to recombinant (Lane 3) or serum-derived (Lane 4) major HBsAg in the presence of DSS resulted in an additional labelled protein complex with a molecular weight of approximately 90 kD. Lane 1 (recombinant HBsAg) and Lane 2 (serum-derived HBsAg) represent control experiments in the absence of crosslinker.
   *Right panel.* Autoradiogram of SDS-PAGE analysis of crosslinking experiments using radiolabelled endonexin II and unlabelled endonexin II (Lane 1), BSA (Lane 3), Asialofetuin (Lane 4) or HBsAg (Lane 2) in solution respectively. Only in the experiment using major HBsAg, an additional band with an apparent MW of 90 kD was observed on SDS-PAGE. Bands with a MW of 68 kD represent a dimer of human liver endonexin II. This dimer was not seen on PAGE, when cross-linking experiments were performed using HBsAg immobilized on solid phase.

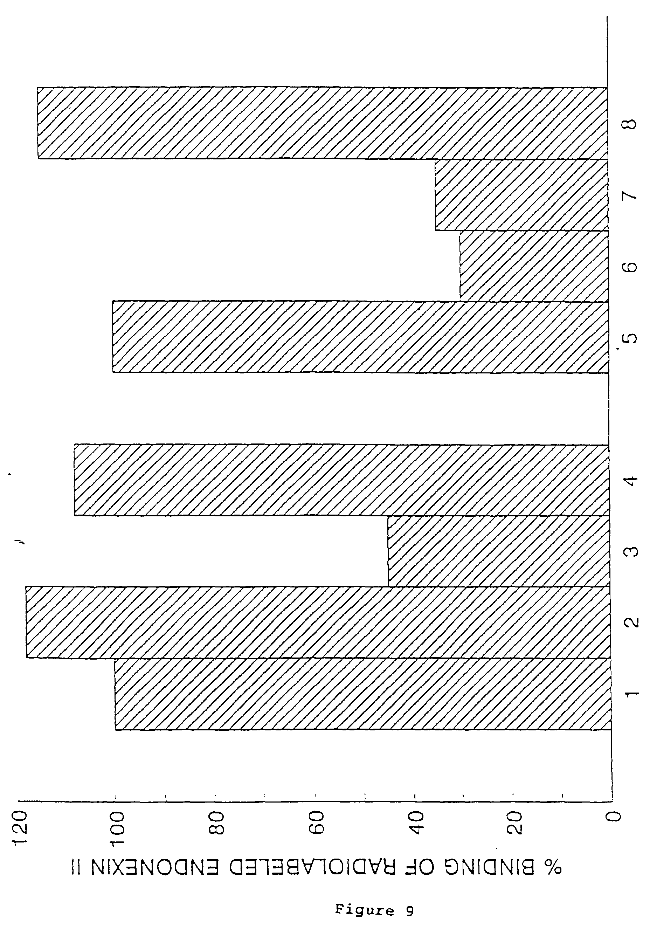

Figure 9 Binding of 15 ng of radiolabelled human liver endonexin II to human (Bars 1 to 4) and rat (Bars 5 to 8) hepatocytes in suspension as percentage of control (Bar 1 and 5). Bars 2-4 and 6-8 express the binding of radiolabelled human liver E-II in the presence of excess (2 µg) unlabelled endonexin II (Bars 2 and 6), excess (2 µg) unlabelled major HBsAg (Bars 3 and 7) and excess (2 µg) unlabelled BSA (Bars 4 and 8).

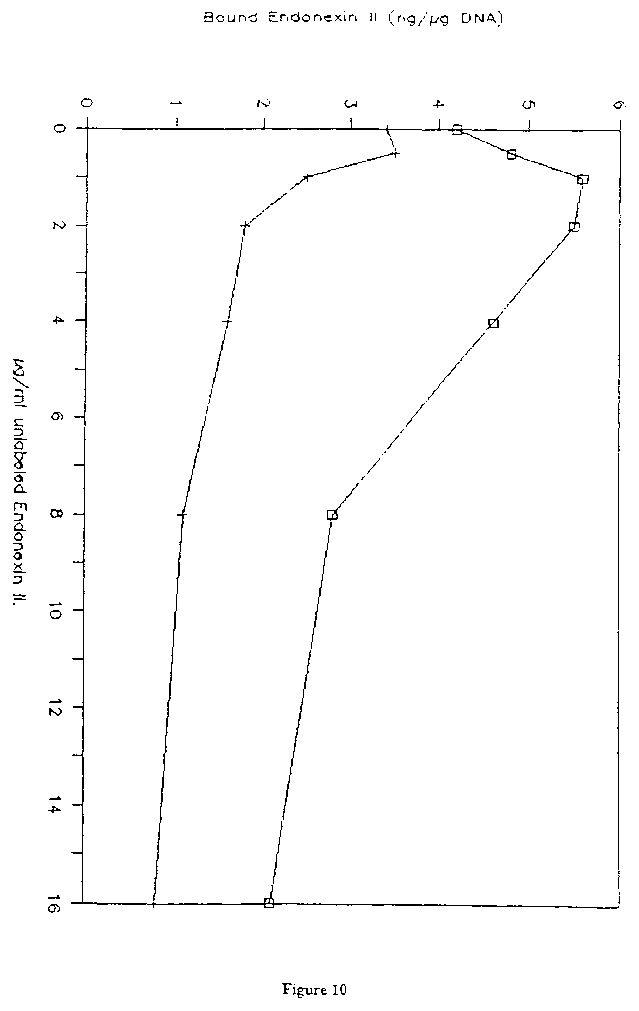

Figure 10 shows the binding of radiolabelled human endonexin II to human (squares) and rat (+) hepatocytes as percentage of control in the presence of varying concentrations of unlabelled human endonexin II.

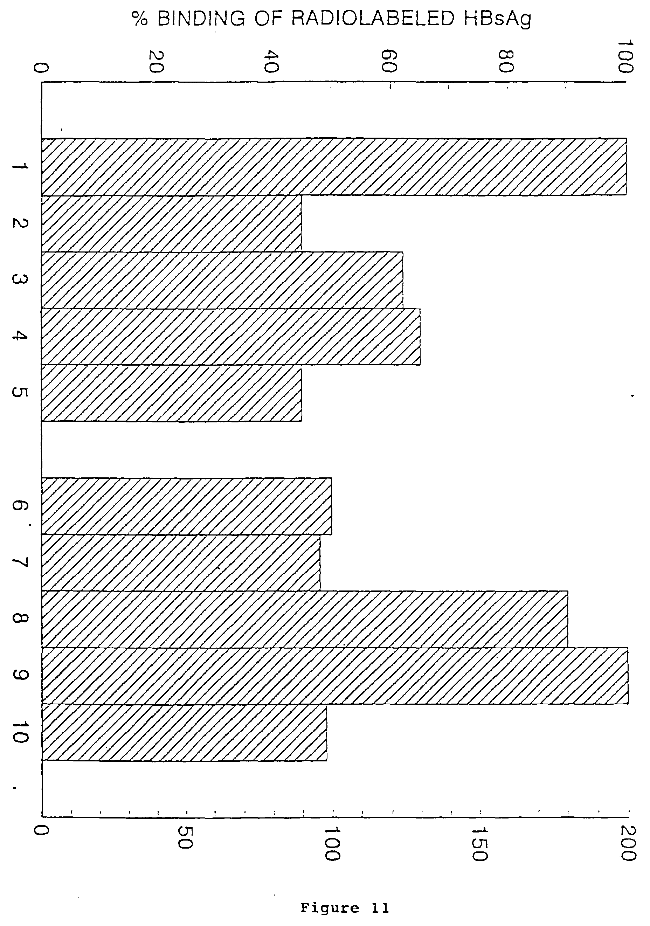

Figure 11 Binding of radiolabelled major HBsAg to intact human (Bars 1 to 5) and rat (Bars 6 to 10) hepatocytes (50.000) in the presence and absence of competitors. Bar 1 (1500 cpm) and 6 (500 cpm) represent binding of radiolabelled HBsAg in the absence of competitor. Bar 2 (600 cpm) and 7 (480 cpm) represent binding in the presence of excess (2 µg) unlabelled HBsAg. Bar 3 (900 cpm) and Bar 8 (900 cpm) represent binding of radiolabelled HBsAg when hepatocytes were first incubated with radiolabelled HBsAg prior to the addition of excess (7 µg for human hepatocytes, 2 µg for rat hepatocytes) unlabelled human liver E-II. Bar 4 (960 cpm) and 9 (1000 cpm) represent the results when radiolabelled HBsAg and excess (7 µg, 2 µg) unlabelled human liver E-II were preincubated prior to addition to the cells. Bars 5 (600 cpm) and Bar 10 (500 cpm) represent the results when hepatocytes were incubated with excess (7 µg, 2 µg) E-II prior to addition of radiolabelled HBsAg to the cells. Although binding is presented as percentage, it must be noticed that there remains a significant difference between human and rat hepatocytes for binding of radiolabelled HBsAg. E-II represents endonexin-II.

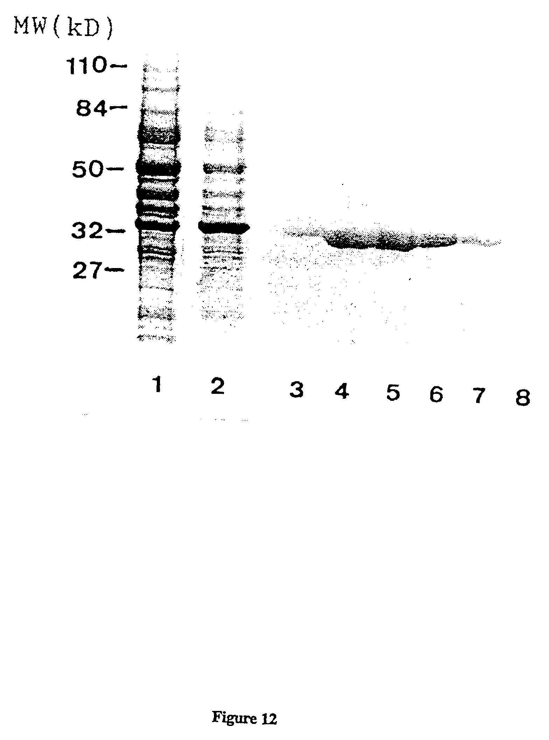

Figure 12 SDS-PAGE analysis of yeast-expressed endonexin II. Cells were harvested, broken down and centrifuged. Lane 1 shows the presence of large amounts of a 34 kD protein (endonexin II) in the cytoplasmatic fraction. After EGTA treatment and recentrifugation of the cell pellets, membrane-associated endonexin-II is also released the supernatant (Lane 2). Lane 3 to 8 show fractions that were eluted from a DE-52 cellulose ion-exchange column.

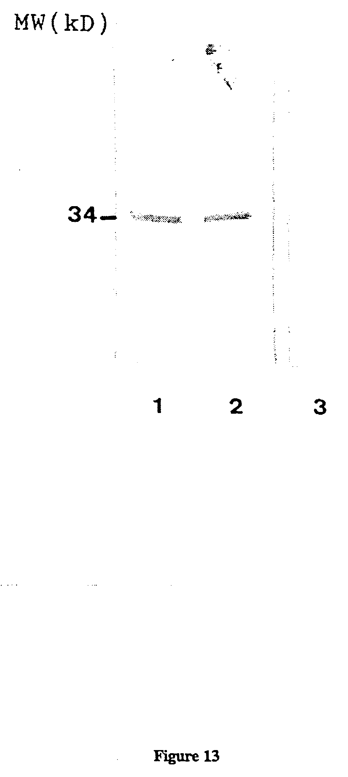

Figure 13 Western blot analysis of purified human liver endonexin II (Lane 1) and recombinant endonexin-II (Lane 2). Detection was performed with rabbit anti-human endonexin II antibodies and a peroxidase conjugated swine ant-rabbit antibody.
   Preimmune serum of the same rabbit did not react with recombinant endonexin II (Lane 3).

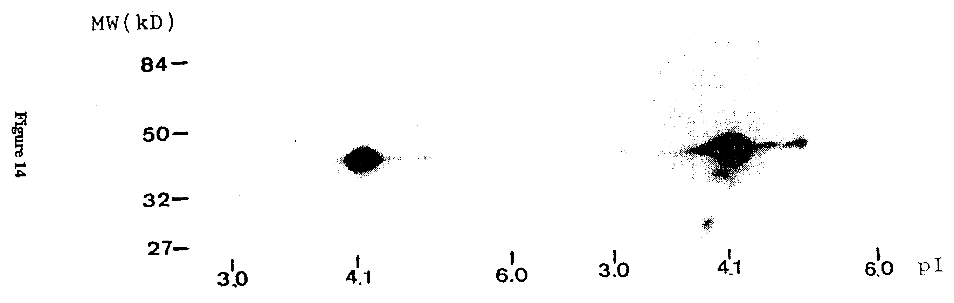

Figure 14 Autoradiogram of IEF/SDS-PAGE analysis of radiolabeled human liver endonexin II (left panel) and recombinant yeast-derived endonexin II (right panel). No differences in molecular weight (34 kD) or iso-electric point (4.1) can be demonstrated.

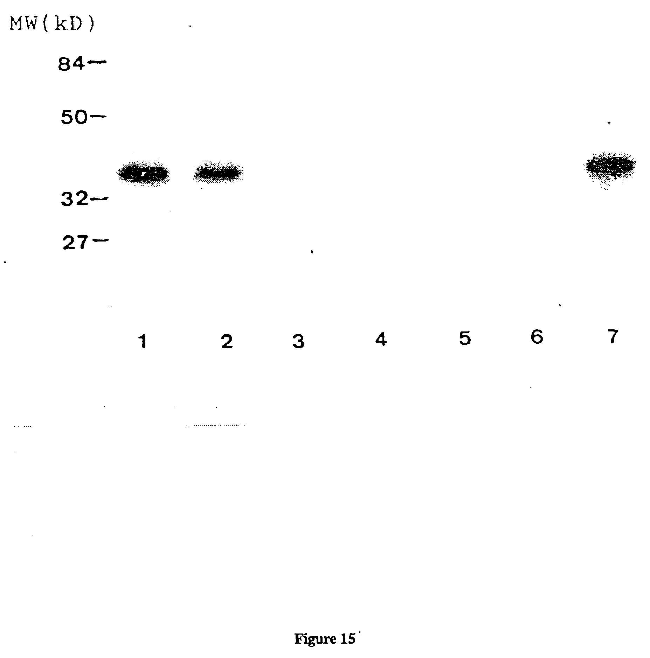

Figure 15 Autoradiogram of SDS-PAGE analysis of human liver (Lane 1) and recombinant endonexin II (Lane 2) bound to solid phase major HBsAg. Radiolabeled recombinant endonexin II was added in the presence of excess unlabeled major HBsAg (Lane 3) or unlabeled human liver endonexin II (Lane 4) to determine specificity of binding. No binding of recombinant endonexin II was observed in this cases, neither after preincubation with anti-endonexin II antibodies (Lane 5), or when Bovine Serum Albumin was immobilized on solid phase (Lane 6). Preincubation of the radiolabeled protein with pre-immune serum did not affect binding to HBsAg (Lane 7).

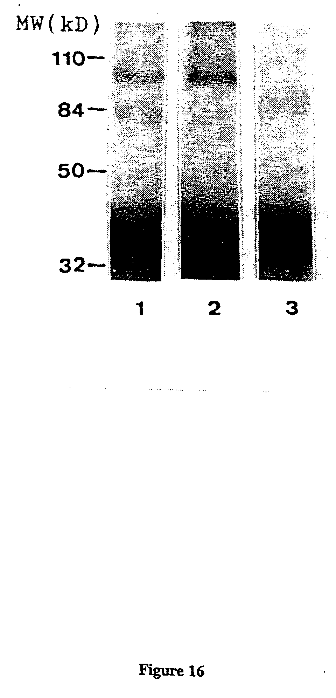

Figure 16 SDS-PAGE analysis of cross-linking experiments between radio labeled human liver (Lane 1) or recombinant endonexin II (Lane 2) and solid phase major HBsAg. Cross-linking resulted in a specific labelled complex with a molecular weight of approximately 90 kD. Lane 3 represents control experiments without cross-linker.

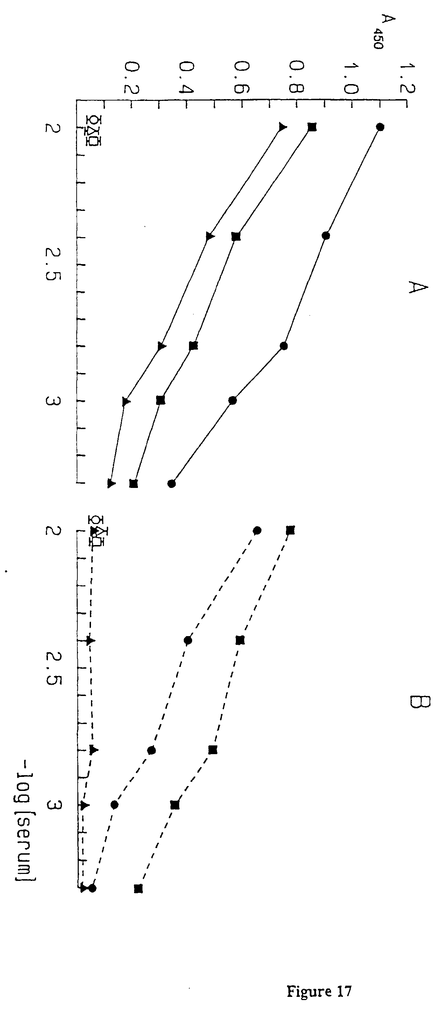

Figure 17 Anti-endonexin II (panel A) and anti-HBs (panel B) response in pre-immunized rabbits (open circle, open square, open triangle) and in rabbits immunized with human liver endonexin II (● native, ■ recombinant) or rat liver endonexin II (black △) as determined by ELISA.

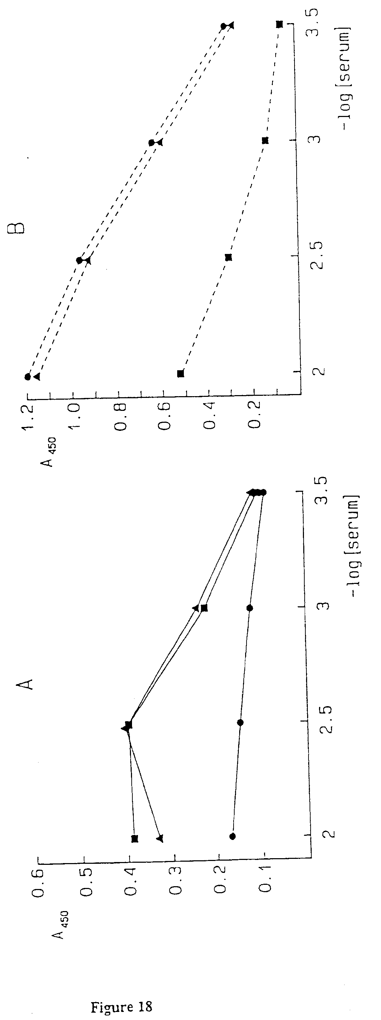

Figure 18 Specificity of anti-HBs (Panel B) and anti-endonexin II (Panel A) activity in serum of rabbit immunized with human liver endonexin II. Panel A: when antiserum was incubated with major HBsAg (●-●), with endonexin II (■-■) or with BSA (black △-△) respectively prior to ELISA using major HBsAg immobilized on solid phase. Panel B: similar experiments when antiserum was incubated with human liver endonexin II (■--■), with HBsAg (●--●) or with BSA (△--△) respectively prior to ELISA using human liver endonexin II immobilized on solid phase.

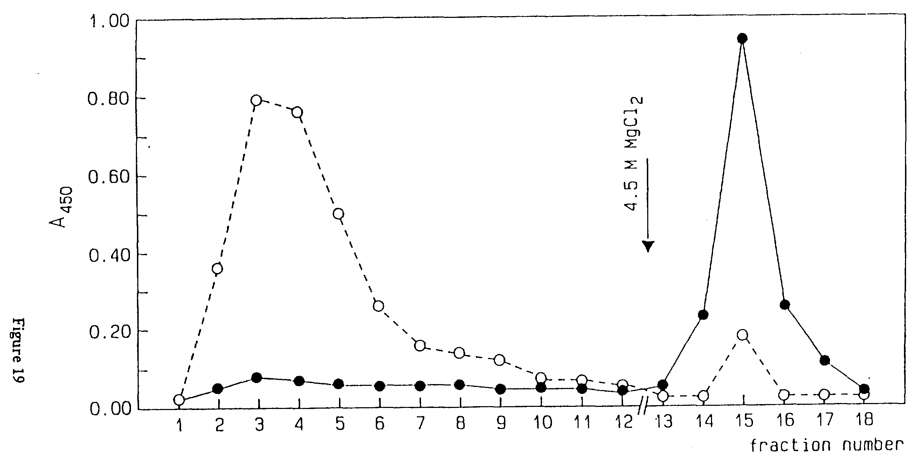

Figure 19 Separation of antibodies, reacting with major HBsAg or recombinant endonexin II, by affinity chromatography on purified recombinant endonexin II, coupled to CNBr-activated Sepharose. Anti-HBs reactivity (o--o) was predominantly found in the flow-through fractions (1-6), while anti-endonexin II reactivity (●-●) was exclusively found in the eluted fractions (14-17) as determined on ELISA.

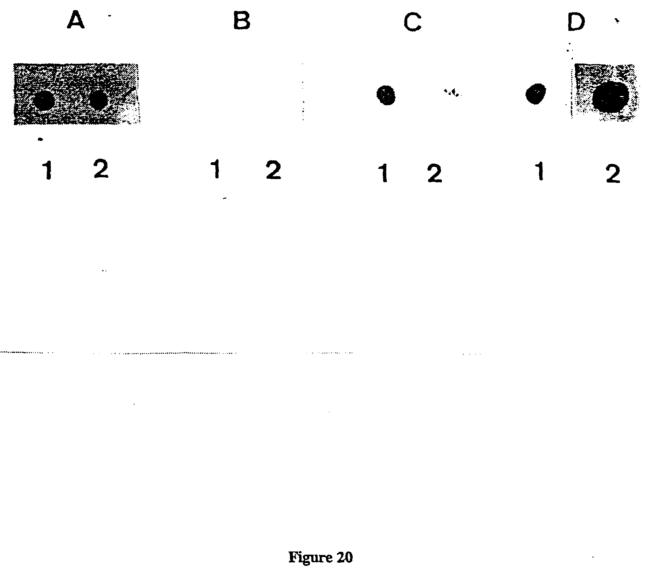

Figure 20 Reactivity of serum of rabbit immunized with human liver endonexin II on immuno dot-blot. Panel A: recombinant HBsAg (1) and human liver endonexin II (2). Panel B: pre-immune serum of the same rabbit. Panel C: after separation of antibodies using affinity chromatography (endonexin II column), anti-endonexin II activity was captured out.
   Panel D: Affinity-purified anti-HBs antibodies (Ab2) recognize recombinant (1) as well as serum-derived major HBsAg (2).

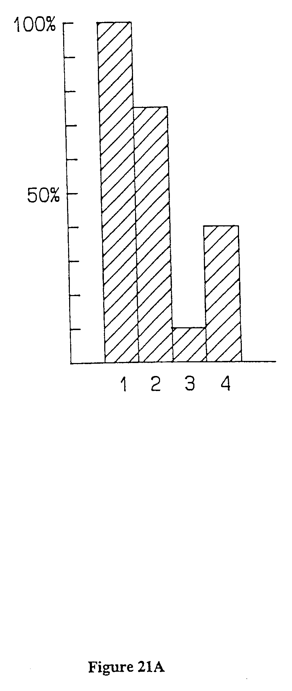

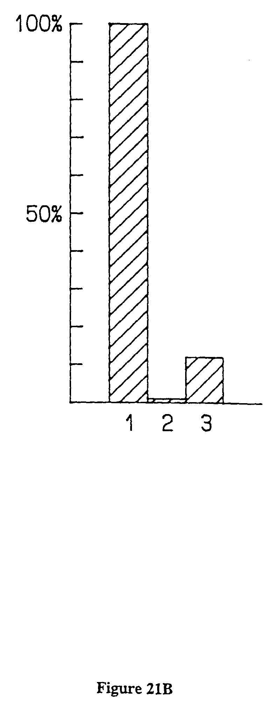

Figure 21 *Left panel*: Immunoprecipitation of radiolabelled major HBsAg by serum of rabbit immunized with human liver E-II. Lane 1 represents control immunoprecipitation of radiolabelled major HBsAg with sheep anti-HBs antibodies. Immunoprecipitation of radiolabelled major HBsAg with affinity purified anti-HBs (AB2) antibodies (Lane 2), with pre-immune serum (Lane 3) or with affinity purified rabbit anti-HBs (Ab2) in the presence of excess of unlabelled HBsAg (Lane 4) is represented as a percentage of immunoprecipitation with polyclonal anti-HBs antibodies.
   *Right panel*: Immunoprecipitation of radiolabelled human liver endonexin II by rabbit anti-E-II antibodies. Lane 1 represents immunoprecipitation of radiolabelled human liver E-II with affinity purified anti-E-II antibodies. Immunoprecipitation of radiolabelled human liver E-II with pre-immune serum (Lane 2) or with anti-human liver E-II in the presence of excess unlabelled human liver E-II (Lane 3) is represented as a percentage of immunoprecipitation using purified anti E-II antibodies. E-II refers to endonexin II.

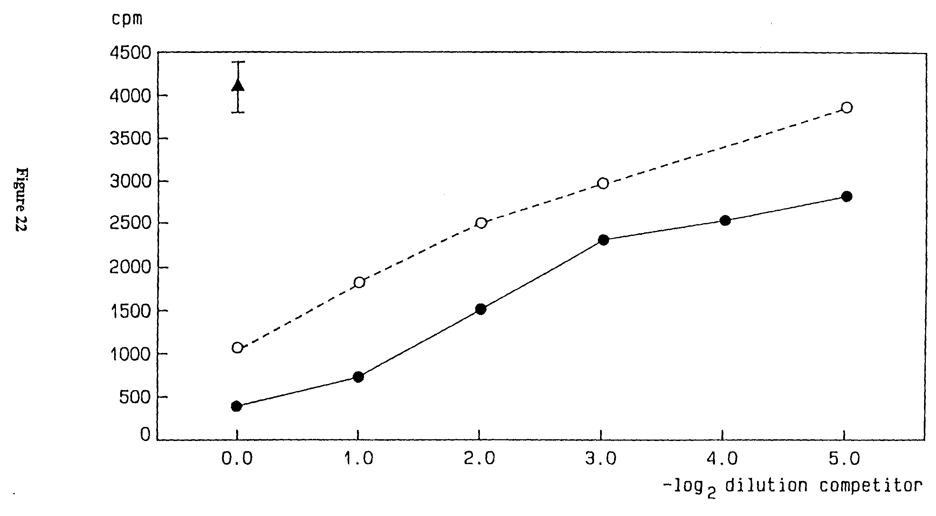

Figure 22 Competition between radiolabelled and unlabelled HBsAg for binding to anti-idiotypic antibodies and between anti-idiotypic antibodies and human liver E-II for binding to radiolabelled major HBsAg. Pre-incubation of anti-idiotypic antibodies on solid phase with serial dilutions of unlabelled HBsAg (o--o), prevents binding of radiolabelled HBsAg (cpm) to the anti-idiotypic antibody. When radiolabelled HBsAg is pre-incubated with serial dilutions of E-II (●-●) in the presence of Ca²⁺ and Mg2+ a comparable inhibition for binding of radiolabelled HBsAg to the anti-idiotypic antibody was observed. Average cpm bound in control experiments are represented by a black diamond crossed by a vertical bar.

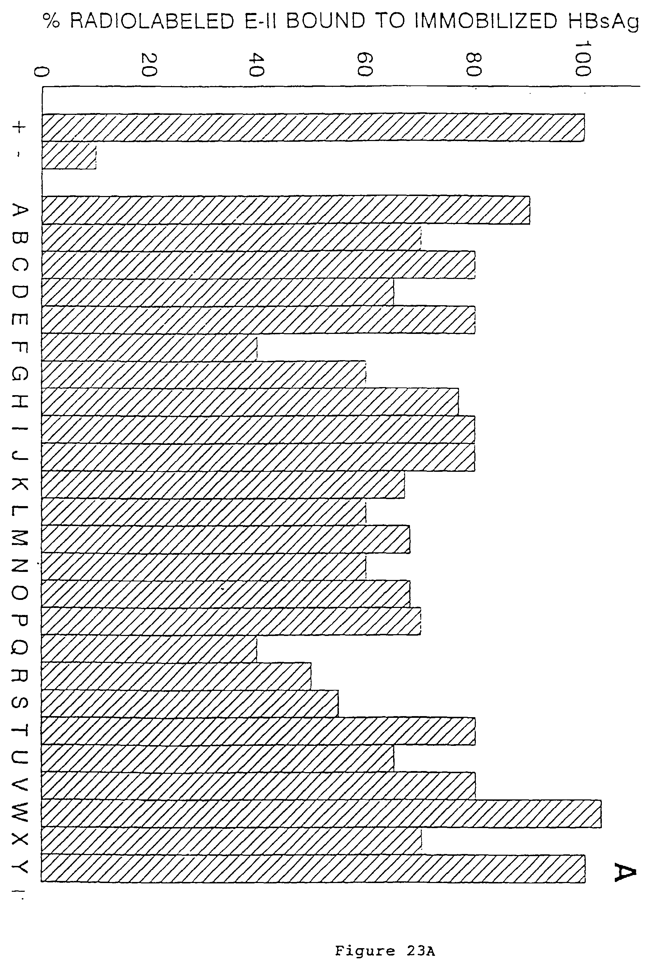

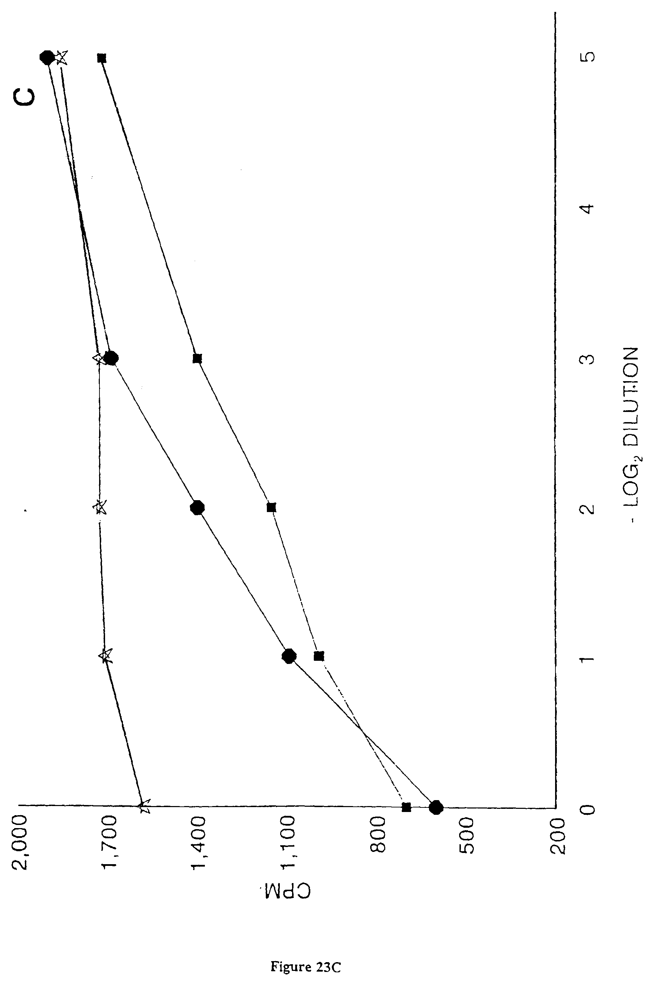

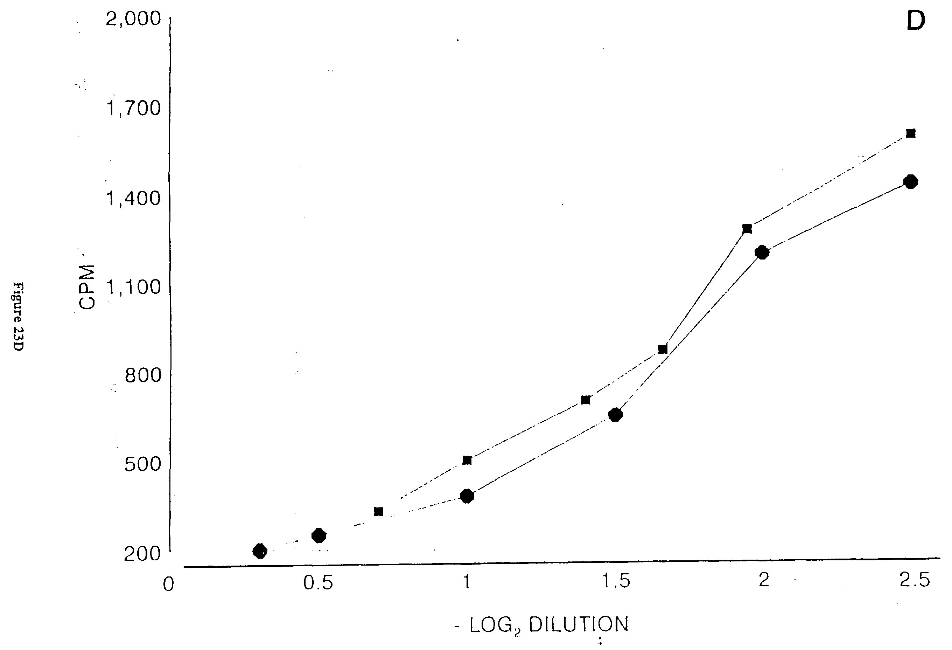

Figure 23A F-peak inhibition assay : Binding of radiolabeled human liver E-II to major HBsAg, immobilized on solid phase, in the presence of peptide fractions obtained by reversed phase HPLC of hydrolysed human liver E-II. Lane (+) and (-) represent binding of radiolabeled E-II in the absence and the presence of intact human liver E-II respectively. Lanes (A) to (Y) represent binding of radiolabeled E-II in the presence of the respective HPLC separated fraction. E-II refers to endonexin II.

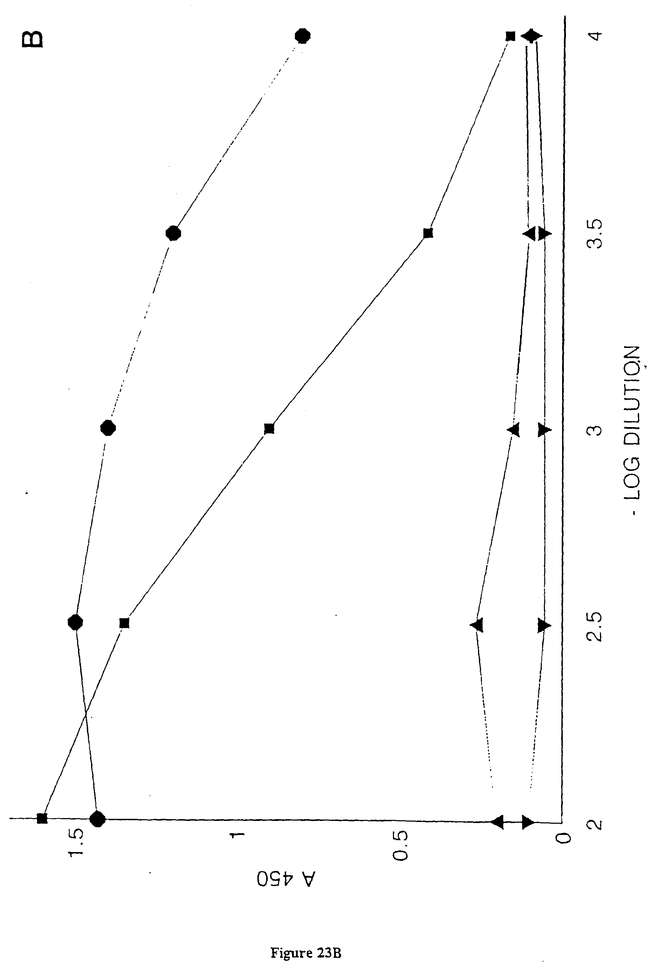

Figure 23B Development of anti-HBs activity in chicken immunized with Fab fragment of rabbit anti-E-II IgG.
   Anti-HBs (Ab2) (■) and anti-rabbit IgG (●) activity of yolk immunoglobulins (IgY) from eggs of chicken immunized with Fab fragment of rabbit anti-E-II pre-immune yolk immunoglobulins. For these assays, HBsAg and rabbit IgG respectively were immobilized on solid phase. Absorbances are read at A450nm. E-II refers to endonexin II.
Figure 23C Competition between radiolabeled Ab2 (cpm), generated by native E-II (Ab2/E-II), and other Ab2's for binding to major HBsAg.
   Binding of radiolabeled Ab2 (cpm), generated by immunization of rabbits with E-II (Ab2/E-II), to major HBsAg on solid phase in the presence or absence of Ab2 generated by the immunization of rabbits with synthetic F peptide or chicken anti-HBs IgG occured after immunization with Fab fragment of rabbit anti-E-II IgG. Pre-incubation of immobilized major HBsAg with serial dilutions of Ab2/E-II (■) or Ab2/F (●) (generated by synthetic F-peptide), prevents binding of radiolabeled Ab2/E-II to major HBsAg. Irrelevant rabbit IgG (△) does not prevent binding. E-II refers to endonexin II.
Figure 23D Pre-incubation of major HBsAg with Ab2/Fab (■) (generated by rabbit anti-E-II IgG), or binding in the presence of serial dilutions of major HBsAg (●), also prevents binding of radiolabeled Ab2/E-II (cpm) to major HBsAg. E-II refers to human liver endonexin II.

### EXAMPLES

### MATERIALS AND METHODS

### Isolation of human liver plasma membranes

Human liver tissue was obtained from post mortem organ donors. The human liver tissue was kept at 4°C, cut into 1-cm³ pieces, snap-frozen in liquid nitrogen, and stored at -80°C until processing. Isolation of plasma membranes was done according to the method of Hubbard et al. (1983) with minor modifications as described by Leenders et al. (1990).

Briefly, 30 g of liver tissue was thawn at 4°C in 0.25 M STM-buffer (0.25 M) and homogenized in a Waring-Blendor followed by a Dounce homogenization. The homogenate was filtered through a 100-mesh nylon filter, the filtrate was adjusted to 20%(w/v) with 0.25 M STM buffer and centrifuged at 300 g (4°C) for 10 min. The pellet was resuspended in a minimal volume of 0.25 M STM-buffer, homogenized in a Dounce homogenizer and centrifuged again for 10 min. at 300 g. Both supernatants were pooled and centrifuged at 1500 g (4°C) for 10 min. The pellet was resuspended in a minimal volume of 0.25 M STM-buffer and 2 M STM buffer (2 M sucrose; 5 mM Tris pH 7.2; 1 mM MgCl₂; 0.1 mM PMSF) was added until a density of 1.18 g/ml was reached. To obtain twice the initial volume of the suspension, 1.42 M STM-buffer (1.42 M sucrose; 5 mM Tris pH 7.2; 1 mM MgCl₂; 0.1 mM PMSF) was added.

Four milliliters of 0.25 M STM-buffer were layered on 25 ml of the suspension and subjected to centrifugation, using a SW 27 rotor, at 82000g (4°C) for 1 h. The plasma membranes, floating on the interphase, were collected, brought to a density of 1.05g/ml with 0.25 M STM-buffer and pelleted at 20000g (4°C) for 15 min, using a Ti50.2 rotor. The pellet was resuspended in a minimal volume of 0.25 M STM-buffer, frozen in liquid nitrogen and stored at -80°C until use.

### Extraction of plasma membrane proteins

Proteins were extracted from plasma membranes using the non-denaturing detergent CHAPS (Hjelmeland, 1980) (3-[(3cholamidopropyl)dimethylammonio]-1-propanesulfonate ) (Bio-Rad, Richmond, CA, USA).

The plasma membrane suspension was treated with an equal volume of CHAPS-extraction buffer (40% glycerol; 0.2M potassium phosphate buffer pH 7.2; 20mM CHAPS) for 30 min at room temperature. The supernatant, obtained by centrifugation at 10.000 rpm for 10 min., was dialysed overnight (4°C) against phosphate-buffered saline (PBS).

The protein concentration of the extract was measured according to Bradford (Bradford, 1976), using the Bio-Rad Protein Assay.

### Radiolabelling of the plasma membrane proteins

Two micrograms of CHAPS-extracted plasma membrane proteins were labelled with 1.37x10⁶ Bq Na¹²⁵I (Amersham, Bucks, England) by incubation for 17 min at room temperature with Iodogen (1,3,4,6-tetra-chloro-3α,6α-diphenylglycouril) (Pierce, Rockford, IL, USA) according to the method of Salacinski et al., 1981. The reaction was stopped by adding 100 µl of a 1 M KI solution to the mixture and free iodine was removed by passage of the reaction mixture over a Sephadex G25 column (Pharmacia, Uppsala, Sweden), blocked with 1% BSA (Sigma, St Louis, USA) in PBS. Labelled plasma membrane proteins were stored at 4°C for no longer than 1 week, before use.

The incorporation of ¹²⁵I precipitable protein in each fraction was determined by precipitating 5 µl of these fractions with 25% trichloro-acetic acid (TCA) (Janssen, Beerse, Belgium) in the presence of 500 µg BSA. Only fractions with a ¹²⁵I precipitability of more than 80% were used for further studies.

### Generation of rabbit polyclonal antibodies against human liver endonexin II

A solution containing 20 µg purified human liver endonexin II was brought to a volume of 1 ml with PBS and was mixed intensively with 1 ml Complete Freund's adjuvant (Sigma, St. Louis, MO, USA). Before injecting the antigen, 5 ml of blood was taken from each rabbit and serum was investigated by Ouchterlony immunodiffusion to exclude natural immunity against human endonexin II. Two rabbits were injected subcutaneously four times with 0.5 ml of the antigen-containing solution (20 µg human liver endonexin II/rabbit for four injections). After two booster injections on day 16 and 40, both rabbits showed an immune response to endonexin II as demonstrated by Ouchterlony immunodiffusion.

### Enzyme linked Immunosorbent Assay (ELISA).

100ng of each antigen was immobilized on high bond ELISA dishes (Greiner, Labortechnik, Nürtingen, Germany) by overnight incubation at 4°C. After blocking the non-specific binding sites, by incubating the wells with Phosphate buffered saline (PBS)/Blotto 1%, wells were incubated with serial dilutions of antibody, followed by incubation with peroxidase conjugated swine anti-rabbit antiserum (Dakopatts A/S, Glostrup, Denmark). The reaction product was developed with the use of tetramethylbenzidine (TMB) (Boehringer, Mannheim, Germany) in the presence of hydrogen peroxide and optical densities were measured at 450nm.

### Immuno-dot blot analysis.

One microgram of each antigen in PBS was spotted on a nitro-cellulose filter (Schleicher and Schuell, Dassel, Germany). Non-specific binding sites were blocked by incubating the filters with PBS/Blotto 1%. Subsequently, filters were incubated with a 1:500 dilution of rabbit antiserum. After washing with PBS/Tween 20 0.05%, filters were incubated with peroxidase conjugated swine anti-rabbit antiserum (Dakopatts A/S). The reaction product was developed with the use of 0.06% diaminobenzidine (Sigma) in 50mM Tris (pH 7.5) in the presence of hydrogen peroxide.

### Purification and separation of antibodies.

Antibodies were intially purified by protein A-Sepharose (Pharmacia, Uppsala, Sweden) column chromatography. The resulting IgG protein (5mg) was subsequently applied to an affinity column, prepared by the coupling of purified recombinant human liver endonexin II to CNBr-activated Sepharose (Pharmacia). Bound antibodies were eluted with 4.5 M MgCl₂.

### Radiolabeling of major HBsAg.

Two microgram of protein was labeled with 1.37x10⁶ Bq Na¹²⁵I (Amersham, Buckinghamshire, England) by incubation with Iodogen (1, 3, 4, 6-tetrachloro-3a, 6a diphenylglycouril) (Pierce, Rockford, IL, USA) for 17 min. at room temperature according to the method of Salacinski et al. (1981). Free Iodine was removed by passage of the reaction mixture over a Sephadex G25 column (Pharmacia) blocked with 1% Bovine serum albumin (BSA) (Sigma) in PBS. The specific activity amounted to 1.7.10⁷ cpm/µg HBsAg. Recombinant major HBsAg was a kind gift of Dr. W. J. Miller (Analytical Research and Development, Merck Sharp and Dohme Laboratories, West Point, PA, USA; McAleer et al., 1984). Serum derived major HBsAg was a kind gift of Dr. N. Lelie (Central Laboratory of the Netherlands Red Cross Blood Transfusion Service, Amsterdam, The Netherlands; Brummelhuis et al., 1981).

### Isolation of adult human hepatocytes

Human hepatocytes were isolated from human liver tissue obtained from post-mortem donors by two-step collagenase perfusion as described previously (Rijntjes et al., 1986; Moshage et al., 1988). Viability of the isolated cells as determined by trypan blue exclusion assay was 90%. Cells were cultured on 24 well plates (200.000 cells/well) containing coverslips coated with extracellular matrix (ECM). Cells were cultured in hormonally defined medium supplemented with 1.5% DMSO. Three days after isolation, cells were used for binding experiments. Prior to the bindings assay cells were incubated with ice cold culture medium containing 1 mM Ca²⁺ and 1 mM Mg²⁺ and 1% BSA to reduce non-specific binding.

### Peptide synthesis

All of the peptides described were synthesized on Tentagel S-RAM (Rapp Polymere, Tübingen, Germany), a polystyrene-polyoxyethylene graft copolymer functionalized with the acid-labile linker p[5-alpha-1(9H-fluoren-9-yl)methoxyformamido-2,4-dimethoxybenzyl]-phenoxyacetic acid (Rink et al., 1987) in order to generate peptide carboxy-terminal amides upon cleavage. Tertio-butyl-based side chain protection and Fmoc-a-amino-protection was used. Couplings were carried out using preformed O-pentafluorophenyl esters. All syntheses were carried out on a Milligen 9050 PepSynthesizer (Novato, Ca, USA) using continuous flow procedures. Following cleavage with trifluoroacetic acid in the presence of scavengers and extraction with diethylether, all peptides were analysed by C18-reverse phase chromatography.

### EXAMPLE 1: Binding of radiolabelled plasma membrane proteins to immobilized recombinant major and large HBsAg (rHBsAg)

Recombinant major HBsAg (McAleer et al., 1984) or serum-derived HBsAg (Brummelhuis et al., 1981) was used to coat high bond ELISA dishes (1 µg/well) (Greiner Labortechnik, Nurtingen, Germany). Non-specific binding sites were reduced by incubating the wells with a 3% gelatin (Merck, Darmstadt, Germany) solution in PBS for 2 h at room temperature. After washing with PBS-0.01% Tween 80, radiolabelled plasma membrane proteins (800,000 cpm) were added in the presence of 1 mM Ca²⁺ and 1 mM Mg²⁺. After incubation for 1 h at room temperature, the wells were washed with PBS-0.01% Tween 80 -1 mM Ca²⁺ -1 mM Mg²⁺ until no more radioactivity was found in the washing solutions. Subsequently, 40µl of a solution containing 9M Urea (Janssen, Beerse, Belgium) and 1% Triton X-100 (Bio-Rad) was added to the wells. After overnight incubation at 4°C, the eluates of the wells were pooled and stored at -80°C until use.

Purity of the eluted protein was assessed by SDS-PAGE and by two-dimensional IEF/SDS-PAGE. As shown in Figure 1, one protein band with an apparent molecular weight of 34 kD was observed on SDS-PAGE, while an analysis on two-dimensional IEF/SDS-PAGE indicated that only one single protein (34 kD, pI 4.1) was bound by major HBsAg (Figure 2B).

In control experiments using BSA-coated microtiter plates, or in control experiments performed in the presence of an excess of unlabelled plasma membrane protein (Figure 1) or major HBsAg (data not shown), no radiolabelled proteins were bound to the ELISA dishes. In addition, when radiolabelled plasma membrane proteins from rat liver were used, a negative result was obtained.

A similar binding experiment was carried out, in which 1 µg recombinant major HBsAg (McAleer et al., 1984), as well as an equimolar amount of recombinant large HBsAg (McAleer et al., 1984) were used to coat microtiter plates as described above. The addition of radiolabelled plasma membrane proteins under the conditions as described above resulted in the detection of a 34 kDa radiolabelled plasma membrane protein bound to recombinant major, as well as to recombinant large HBsAg. It is, however, important to note that large HBsAg seems to bind more efficiently to radiolabelled proteins than does major HBsAg.

### EXAMPLE 2: Isolation and two-dimensional gelelectrophoresis of 34 kD proteins

### Isolation of the major HBsAg-binding 34 kD fraction

A large-scale isolation of the 34 kD protein fraction from CHAPS-extracted proteins of human liver plasma membranes (see Figure 1 and Materials and Methods) was performed as described below.

A protein fraction with an apparent molecular weight of 34 kDa was isolated either from horizontal, precast SDS-PAGE gradient gels (ExcelGel; Pharmacia) or from vertical 1 mm 10% Tricine (Janssen) (Schägger & Von Jagow, 1987) gels or from the Model 491 Prep Cell system (Bio-Rad). In the former cases the protein was electroeluted from the excised 34 kDa band in 0.1% SDS-192 mM glycine by using the S&S Biotrap (Schleicher and Schuell, Dassel, Germany). In the latter case the 34-kDa proteins were recovered in elution buffer (0.1% SDS-192 mM glycine) and the appropriate fractions were pooled. The eluted fractions were snap-frozen in liquid nitrogen and stored at -80°C until use.

Prior to two-dimensional gel electrophoresis, the 34 kDa protein fraction was concentrated 10-fold by vacuum centrifugation in a Savant speedvac or by Centriprep filtration (cut-off: 10 kDa; Amicon Division, W.R. Grace & Co., Beverly, MA, USA), precipitated overnight (4°C) with TCA (20% w/v final concentration) and washed with 20% TCA, acetone and acetone containing 1% (v/v) triethylamine. The sample was dried under reduced pressure and dissolved in no more than 200 µl lysis buffer (9.5 M Urea, 5% 2-mercaptoethanol, 2% CHAPS and 5% Resolyte pH 3.5-10 (Hoefer, San Fransisco, CA, USA)).

### Two-dimensional gelelectrophoresis of 34 kD fraction

Isoelectric focussing (IEF)/SDS-PAGE was based on the system of O'Farrel et al. (1977) with the following modifications as described by Meheus et al. (1987). The first dimension was performed on ultrathin (0.5 mM) polyacrylamide gels coated onto PAG-Gelbond film (FMC, Rockland, ME, USA) and with a pH gradient built up by a mixture (3:1) of Pharmalyte pH 2.5-5 (Pharmacia) and Resolyte pH 4-8 (Hoefer).

The IEF gel was prerun (500 V for 1 h) prior to the application of the sample and run for 6250 Vh (500 V for 0.5 h, 1000 V for 2 h, and 2000 V for 2 h). After electrophoresis in the first dimension, the gels were fixed in 10% (w/v) TCA for 30 min, washed overnight with 40% methanol and stained in 0.1% (w/v) Coomassie Brilliant Blue R-250 (Merck) in 10% (v/v) acetic acid-50% (v/v) methanol.

For electrophoresis in the second dimension, the appropriate lanes were cut out, washed with distilled water (10 min), 50 mM Tris-HCl pH 6.8 (10 min) and equilibrated against 4% SDS-100 mM DTT-50 mM Tris-HCl pH 6.8 (30 min). Electrophoresis in the second dimension was performed in a 10% Tricine gel (Schägger & Von Jagow, 1987) and stained with Commassie Brilliant Blue R-250.

Although the protein fraction obtained above showed a single protein band of 34-kDa on one-dimensional gel electrophoresis, several proteins with approximately the same molecular weight were seen on two-dimensional gel electrophoresis (Figure 2A). The spot of interest on two-dimensional gel electrophoresis was identified by analysis of a mixture of radiolabelled major HBsAg-binding protein, obtained from the binding assays (Example 1), and the unlabelled 34 kD fraction after staining and autoradiography. Comigration of labelled and unlabelled material resulted in a Coomassie stainable spot, coinciding with a single radioactive protein spot with a molecular weight of 34-kDa and a pI of 4.1. (Figures 2A (arrow) and 2B).

### EXAMPLE 3: Sequencing of the 34-kDa major HBsAg-binding protein

The protein spots of interest were cut out from the two-dimensional gels, concentrated into one spot using the method described by Rasmussen et al. (1991) and stained with 0.1% Ponceau S in methanol/acetic acid/water (50:10:50).

A sequenceable amount of this 34 kD protein (estimated at 4 µg) was collected from 56 two-dimensional IEF-SDS/Tricine gels. The concentrated spot was cleaved into peptides according to the method of Vanfleteren et al. (1992). Briefly, after dissociation of the dye, the protein was hydrolyzed in the gel matrix in 3% formic acid at 112°C for 4 h.

The eluted peptides were separated by reversed phase HPLC on a Vydac C-4 column (2.1 x 250 mm, Hesperia, CA, USA) using a 140B Solvent Delivery System (Applied Biosystems, Foster City, CA, USA) and eluted with a linearly increasing gradient of acetonitrile in 0.1% trifluoroacetic acid. The column was directly connected to a 8-µl flow cell of a 1000 S diode array detector (Applied Biosystems). Peptide peaks were registered on a chart recorder and collected manually. The HPLC elution pattern of these peptide is shown in Figure 3. Finally, the resulting peptides were sequenced using a pulsed liquid model 477A Sequenator (Applied Biosystems) equipped with an on-line 120 phenylthiohydantoine analyser (Applied Biosystems). The amino acid sequence of different peptides is demonstrated in Table 2.

**Table 2**

| Amino acid sequences obtained by sequencing the peaks indicated (arrows) in Figure 3. | |
|---|---|
| peak AH5 | AETLRKAMKGL |
| peak AH21 | YKKALLLL (Q) GE |
| peak AH24a | AQALFQAGEL* |
| peak AH24b | YKKALLLL*GED |
| peak AH24c | TSGDYKKALLLL*GED |
| peak AH25 | ****FQAGEL (K) *G |
| peak AH42 | AYELK (Q) ALKGAGTNEK |

Parenthesis indicate amino acids which gave no clear signal. Asteriks indicate no signal was obtained for this residue.

### EXAMPLE 4: Identification of human endonexin II as the major HBsAg-binding protein

Amino acid sequences obtained from the analysis were screened against the Swiss protein databank (Department of Medical Biochemistry of the University of Geneva and the EMBL data libraries) and resulted in the identification of endonexin II as the major HBsAg binding protein. Comparison with the sequence of endonexin II as reported by Kaplan et al. (1988) shows that peak AH5 corresponds to amino acids 21 to 31, peak AH21 to amino acids 308 to 318, peak AH24a to amino acids 176 to 186, peak AH24b to amino acids 308 to 319, peak AH24c to amino acids 304 to 319, peak AH25 to amino acids 180 to 188, and peak AH42 to amino acids 93 to 108.

Based on the information of the amino acid sequences of peptides AH5, AH21, AH24, AH25, AH42 (Figure 3 and Table 2) and sequence databank searching (Swiss protein data bank), it was found that the 34-kDa protein of the invention unequivocally belonged to the annexin family and could be identified as human endonexin II or annexin V (Kaplan et al., 1988; Walker et al., 1992).

### EXAMPLE 5: Binding of isolated human and rat endonexin II to major HBsAg

Isolation of human and rat endonexin II from the respective liver tissues was from hereinafter done according to method of Haigler et al. (1987) with some minor modifications. In short, 50 g of liver tissue was thawed at 4°C in homogenization buffer (buffer H), which contained 20 mM Hepes (pH 7.4), 150 mM KCl, 2 mM MgCl₂, 1 mM benzamidine and 1 mM phenylmethanesulfonyl fluoride (PMSF). The thawed tissue was homogenized in a Waring-Blendor and the homogenate was centrifuged at 800 g (4°C) for 15 min. The pellet was resuspended in buffer H and centrifuged again. Both supernatants were pooled and filtered through a 100 mesh nylon filter. CaCl₂ was added to the filtrate to a final concentration of 1 mM and the solution was allowed to stand for 30 min. In the presence of Ca²⁺, all calcium-dependent phospholipid binding proteins bind to the plasma membrane (Haigler et al., 1987). After 30 min the solution was centrifuged at 100,000 g (4°C) for 20 min and the supernatant was discarded. The pellet was resuspended in buffer H, supplemented with CaCl₂ by Dounce homogenization and centrifuged at 100,000 g. This step was repeated three times. After the wash steps, the pellet was resuspended in buffer H containing 2 mM EGTA, allowed to stand for 15 min and centrifuged at 100,000 g (4°C) for 20 min. All calcium-dependent plasma membrane proteins are released from the membrane. The supernatant was discarded and concentrated to 15 ml by pressure filtration through an Amicon YM-10 (Amicon, Conn. Beverly, MA, USA) membrane.

The concentrated EGTA eluate was fractionated on a Sephadex G-100 column (2.5 x 90 cm) (Pharmacia, Uppsala, Sweden) equilibrated with ammonium acetate (0.06 M, pH 8.3). The flow rate was 0.5 ml/min and 5 ml fractions were collected. Fractions of interest were concentrated, investigated on 12% SDS-PAGE and adjusted to 5 mM ammonium acetate (pH 8.65). This sample was applied to a DE-52 cellulose (Whatman, Maidstone, England) column (1 x 4 cm), equilibrated with ammonium acetate buffer (5 mM, pH 8.65), and eluted with the following gradient of ammonium acetate (5 mM, pH 8.65); 160 ml of 5-30 mM, 60 ml of 30-75 mM and 125 ml of 75-350 mM. The flow rate was 0.5 ml/min and 5-ml fractions were collected. The peak eluted at ± 300 mM ammonium acetate was concentrated and checked on 12% SDS-PAGE and on two-dimensional gel electrophoresis, using the Immobiline Dry Strip Kit (Pharmacia) in the first dimension and Excel gel (Pharmacia) in the second dimension. Finally, 10 µg of the purified protein was subjected to limited acid hydrolysis and sequenced as described above.

Binding studies were performed with ¹²⁵I-labelled human and rat liver endonexin II under the experimental conditions as described for labelled plasma membrane proteins (see example 1). In this type of binding study, however, the binding was performed by adding 15 ng of ¹²⁵I-labelled human or rat endonexin II to 1 µg of major HBsAg coated on wells of the ELISA dish.

As shown in Figure 4 (lane 1), this resulted in the binding of human liver endonexin II to recombinant major HBsAg. Specificity of the binding was demontrated by the ability of an excess of unlabelled human liver endonexin II (2 µg) or major HBsAg (2 µg) or human plasma membrane proteins (20 µg) to compete for the binding to major HBsAg (Figure 4, lanes 2-4).

Preincubation of labelled human liver endonexin II with rabbit anti-human liver endonexin II IgG (purified as illustrated in Materials and Methods) inhibits the binding of human liver endonexin II to major HBsAg (Figure 4, lanes 5-6). The binding was not inhibited by pre-immune IgG of the same rabbit. Figure 5 represents the results of preincubation of radiolabelled human liver endonexin II with varying concentrations of anti-endonexin II for binding to major HBsAg. Binding experiments with equal amounts of radiolabelled human and rat liver endonexin II (10ng) demonstrated that rat liver endonexin II, in comparison with human liver endonexin II, was hardly able to bind to major HBsAg (Figure 6).

### EXAMPLE 6: Inhibition of major HBsAg binding to intact human hepatocytes by human liver endonexin II

To investigate whether human liver endonexin II is able to inhibit the binding of radiolabelled major HBsAg to intact hepatocytes, radiolabelled major HBsAg and excess unlabelled human liver endonexin II were pre-incubated for 30 min. at 4°C before addition to cultured cells.

Approximately 200.000 cells were incubated for 1 hour at 4°C with 1 ml binding medium (culture medium supplemented with 1mM Ca²⁺, 1mM Mg²⁺ and 1% BSA). Radiolabelled major HBsAg, in the absence or presence of an excess of unlabelled HBsAg (to determine the aspecific background binding), incubated with human liver endonexin II or control protein, was added and cells were incubated at 4°C for 2 hours. Subsequently, cells were washed with cold binding medium until no more radioactivity was found in washing solutions, and lysed with 0.2N NaOH/ 0.5% SDS to allow the determination of bound radioactivity.

As shown in Figure 7, the binding of radiolabelled major HBsAg to cultured human hepatocytes was inhibited by human liver endonexin II.

### EXAMPLE 7: Crosslinking of major HBsAg to human endonexin II

Crosslinking was performed by adding a solution of 50 mM DSS (disuccinimidyl suberate) (Pierce, Rockford, IL, USA) in DMSO at a final concentration of 1 mM to the major HBsAg coated wells containing ¹²⁵I-labelled endonexin II. The reaction was allowed to proceed for 30 min at 4°C and the reaction was stopped by adding 1 M glycine to a final concentration of 100 mM. Finally, the wells were washed as described above and bound proteins were eluted and examined by 12% SDS-PAGE.

When 1 mM DSS was added in binding experiments (as described in Example 1) of ¹²⁵I-labelled endonexin II to immobilized recombinant or serum-derived major HBsAg, an additional band with an apparent molecular weight of 90 kD was seen on SDS-PAGE (Figure 8).

This protein complex can either be the result of:
1) interaction between one molecule endonexin II (MW 34 kDa) and two molecules of major HBsAg (MW 27 kDa) or of
2) interaction between two molecules of endonexin II and one molecule of major HBsAg.

Similar results were obtained when DSS was added to a mixture of ¹²⁵I-labelled endonexin II and major HBsAg in solution, while no such protein complex was found when DSS was added given to a mixture of ¹²⁵I-labelled endonexin II and BSA, asialofetuin (ASF) or unlabelled endonexin II (Figure 8).

Such a protein complex was also not found when radiolabelled rat liver endonexin II and major HBsAg were cross-linked. The specificity of crosslinking was further demonstrated by the absence of a ± 90 kD complex if chemical crosslinkers other than DSS (DMA or dimethyladipimidate, DMP or dimethylpimelimidate, DTBP or dithiobispropionimidate, DTP or dithiopropionimidate) were used. Based on these findings the interaction between major HBsAg and human liver endonexin II can be considered specific and the interaction distance between endonexin II and HBsAg can be estimated to be 11.4 Å (chain length of DSS).

### EXAMPLE 8: Binding of ¹²⁵I-labelled human liver endonexin II to human and rat hepatocytes

To investigate whether endonexin II was able to prevent binding of major HBsAg to intact hepatocytes, it was necessary to determine whether human liver endonexin II itself is capable to bind to these cells. Therefore, human and rat hepatocytes, were incubated with ¹²⁵I-labelled human liver endonexin II.

Approximatively (15 ng) ¹²⁵I-labelled human liver endonexin II was added in the presence of unlabelled excess (2 µg) of human liver endonexin II, unlabelled excess (2 µg) of major HBsAg or unlabelled excess (2 µg) of BSA.

As shown in Figure 9, binding was obtained between human hepatocytes and endonexin II, while a similar binding pattern was not observed using rat hepatocytes. Furthermore, excess major HBsAg was able to prevent binding of ¹²⁵I-labelled human liver endonexin II to intact cells (rat and human), indicating that there is an interaction between HBsAg and human liver endonexin II which leads to a competition of binding between hepatocytes and human liver endonexin II. As a control, BSA was added in the presence of ¹²⁵I-labelled human liver endonexin II. In this case, no inhibition of binding of ¹²⁵I-labelled human liver endonexin II could be demonstrated.

To investigate the difference in binding of ¹²⁵I-labelled human liver endonexin II to human and rat hepatocytes, binding studies were performed on human and rat hepatocytes (50,000 cells) using varying amounts of unlabelled excess of endonexin II. As shown in Figure 10 there is a marked difference between human and rat hepatocytes for binding of ¹²⁵I-labelled human liver endonexin II. In the former case, addition of small amounts of unlabelled human liver endonexin II results in an increased binding of ¹²⁵I-labelled human liver endonexin II, while inhibition of binding occurs when at least 7 µg (final concentration 7 µg/ml) of unlabelled human liver endonexin II was added to the reaction mixture. However, in the latter case (rat hepatocytes) inhibition of binding of ¹²⁵I-labelled human liver endonexin II was observed after addition of 1 µg of unlabelled human liver endonexin II (final concentration 1 µg/ml). No increase of binding was observed when smaller quantities of unlabelled human liver endonexin II were added.

To investigate whether human liver endonexin II is able to inhibit binding of ¹²⁵I-labelled major HBsAg to intact cells, a certain concentration of free endonexin II has to be present in the medium. As mentioned above, at least 7 µg/ml of human liver endonexin II is needed to prevent the binding of additional ¹²⁵I-labelled human liver endonexin II to 50,000 human hepatocytes. For rat hepatocytes, this amounts to at least 1 µg/ml of human liver endonexin II.

To determine the effect of human liver endonexin II on the binding of major HBsAg to hepatocytes, several experimental protocals were used. Firstly, ¹²⁵I-labelled major HBsAg was incubated with intact hepatocytes (human and rat) for 30 mm at 4°C, before unlabelled excess (7 or 1 µg respectively) human liver endonexin II was added. Secondly, ¹²⁵I-labelled major HBsAg and unlabelled excess human liver endonexin II were mixed for 30 min at 4°C before addition to the cell suspension. Thirdly, unlabelled excess human liver endonexin II was incubated first with intact hepatocytes for 30 min at 4°C before ¹²⁵I-labelled major HBsAg was added. In all three cases, as shown in Figure 11, the addition of human liver endonexin II inhibits the binding of ¹²⁵I-labelled major HBsAg to human hepatocytes. In contrast, using rat hepatocytes, an increased binding of ¹²⁵I-labelled HBsAg (compared to the low binding of radiolabelled HBsAg in the absence of competitors) was observed when an excess of human liver endonexin II and ¹²⁵I-labelled HBsAg, or ¹²⁵I-labelled HBsAg and subsequently excess human liver endonexin II were added to the hepatocytes. However, when the cells were preincubated with unlabelled excess of human liver endonexin II, no increased binding of major HBsAg was observed.

### EXAMPLE 9: Isolation of human liver endonexin II cDNA clones and expression of recombinant human liver endonexin II in yeast.

### Isolation of endonexin II cDNA clones

A human liver cDNA library was constructed in the EcoRI site of the Lambda Zap vector (Stratagene, La Jolla, California, USA) and used to infect XL1 blue cells (Stratagene). Approximately 6,5 x 10⁵ plaques from the library were spread on NZY plates and incubated overnight at 37°C before duplicate replica's were made on nitrocellulose filters (Schleicher and Schuell, Dassel, Germany). Subsequently, these replica's were screened for endonexin II positive clones, using a standard hybridisation protocol and a synthetic 36-mer oligonucleotide (5'-TTTTTCATTTGTTCCAG CTCCCTTCAAGGCATGTTT-3') probe. The sequence of the probe has been derived from amino acid (and correlated nucleic acid) sequence data obtained from previous analysis of isolated major HBsAg-binding protein (Table 2). A DNA 5'-end labeling system (Promega, Madison, WI, USA) was used to label 100 ng (10 pmol) of probe.

Filters were autoradiographed for 48h using intensifying screens. Plaques giving positive hybridisation signals on both filters were considered positive and used for further screening. Finally, 10 endonexin II positive clones were identified and purified by replating at low density and rescreening plaques with the oligonucleotide probe.

Phage stocks of positive clones were used to infect XL1 blue cells in the presence of the helper phage R 408 (Statagene). By recombination events, this results in the construction of a pBluescript plasmid. Colonies of cells appearing on LB/ampicilline plates contain the pBluescript plasmid with the cDNA insert at the EcoR1 site. To confirm the identity of the endonexin II positive clones, plasmid DNA was isolated from XL1 blue cells by an alkaline lysis miniprep method. This DNA was digested with EcoRI (Boehringer, Mannheim, Germany) in the presence of RNAse A (Boehringer). Based on the digestion pattern, five clones with a 1.6 kb insert and one clone with a 2.2 kb insert, analoguos to the full lenght of human placental cDNA, were selected, run on a 0.8% agarose gel, transferred to Hybond nylon membranes (Amersham, Buckinghamshire, England) and hybridised with the 36-mer oligonucleotide probe. From five clones, four were found positive again. DNA sequencing of these clones using the TaqTrack sequencing system (Promega) revealed start and stop codons in all 1.6 kb clones. Complete sequencing of all clones revealed no differences between the human liver 34 kD protein and the already reported human placental endonexin II cDNA (Kaplan et al., 1988).

### Expression of mature human liver endonexin II in S.cerevisiae

The human liver endonexin II coding sequence was isolated as a 981 bp NcoI/Ecl136II fragment from pBSK(-)h endonexin II clone 5.3.1. en subsequently inserted into the NcoI/EcoRV opened vector pYIG1. This vector, a derivative of pTZ18U (Mead et al., 1986), carries a constitutive *S.cerevisiae* promotor (such as the PGK (phosphoglycerate kinase), GAP (glyceraldehyde-3-phosphate dehydrogenase or MF1α (mating factorα ) promotors) and a *S.cerevisiae* terminator (such as the TRP1, GAP or MF1 terminators). The resulting vector was called pYIG1hEXNII.

In a second step the promotor/human endonexin II coding sequence/terminator module was transferred to the *E.coli/S.cervisiae* shuttlevector pSY1. This vector carries the total *S.cerevisiae* 2µ circle plasmid, *E.coli* pBR322 sequences and a *S.cerevisiae* auxotrophic marker such as URA3, HIS3 or LEU2. This transfer was done by isolating the module as a ± 2600 bp BamHI fragment from vector pYIG1hEXNII and inserting it in the BamHI opened vector pSY1. Vectors were isolated where the module was inserted in the sense and antisense direction. These constructs were called pSY1hEXNIIs and pSY1hEXNIIas.

Plasmid DNA of pSY1hEXNIIs and pSY1hEXNIIas was transformed via the spheroplast formation method (Klebe et al., 1983) in *S.cerevisiae* strain AVB100. Several colonies were grown in YPD medium supplemented with 3 % glucose at 28°C. Samples were taken via fractionation of the yeast cells at several time intervals and analysed via SDS-PAGE and western blotting.

A Coomassie Brilliant Blue stainable induced band (M.W. 34-35 Kd) was visible on SDS-PAGE after 24 hours growth. No decrease in expressionlevel could be observed after 120 hours growth (data not shown).

The identity of the expressed protein could be confirmed by western blotting with a polyclonal rabbit anti-endonexin II serum.

The mature human endonexin II is almost exclusively present in the soluble fraction.

### Purification of recombinant Endonexin II.

Cultured yeast cells were broken down with a Brown homogeniser in homogenization buffer (buffer H), containing 20mM HEPES (pH 7.4) (Boehringer), 150 mM KCl, 2mM MgCl₂, 1mM Benzamidine (Sigma, St. Louis, USA), 1mM Phenylmethanesulfonylfluoride (PMSF) (Sigma) and 0.1mM Pefabloc SC (Boehringer) and were centrifuged at 100.000g (4°C) for 30 minutes to remove cell debris. Supernatants (Sup I) were stored at -80°C untill use. Pellets were homogenised in buffer H, supplemented with 1mM Ca²⁺, and left at 4°C for 15 minutes. After centrifugation at 100.000g (4°C) for 20 minutes, the pellet was homogenised in buffer H, containing 2mM EGTA, left for 15 minutes and recentrifuged at 100.000g. The final supernatant (Sup II) was also stored at -80°C untill use.

Sup I and II were further fractionated on a Sephadex G-100 (Pharmacia) column, followed by a DE-52 cellulose column (Watman, Maidstone, England) as described by Haigler et al. (1987). Protein concentration of purified endonexin II was determined according to Bradford (1976), using the Bio-Rad protein assay.

### Chemical characterization of recombinant human liver endonexin II.

As shown in figures 12 to 14, molecular weight, iso-electric point and anti-endonexin II antibody recognition properties of recombinant endonexin II were found to be identical to those of native human liver endonexin II.

### Characterization of HBsAg-binding properties of recombinant human liver endonexin II.

To confirm functional activity of the recombinant protein, binding of radiolabeled recombinant endonexin-II to major HBsAg was performed as described above for native human liver endonexin major-II.

As shown in figure 15, both proteins bind to major HBsAg but not to BSA. Specificity is demonstrated by competition of binding by adding excess unlabeled major HBsAg, or excess unlabeled native human liver endonexin-II. When recombinant radiolabeled endonexin-II is preincubated with polyclonal anti-endonexin-II antibodies, directed to native human liver endonexin-II, binding to HBsAg is completely inhibited. This is not the case when endonexin-II is preincubated with pre-immune serum of the same rabbit. It can be noticed that the binding of recombinant endonexin-II to HBsAg differs in no way from the binding displayed by the native human liver protein. Further confirmation of HBsAg-binding activity is obtained from cross-linking experiments between major HBsAg and radiolabeled recombinant endonexin-II. The specific complex, with an apparant molecular weight of 90 kD, that is obtained by cross-linking major HBsAg and endonexin-II was obtained in experiments with recombinant endonexin-II, as well as in experiments with native human liver endonexin-II (fig. 16). In control experiments with BSA or unlabeled HBsAg, or in experiments without cross-linker, no high molecular weight complex can be obtained. This confirms that the interaction between HBsAg and endonexin-II is specific and is stabilised by the addition of a chemical cross-linker.

### EXAMPLE 10: Anti-idiotypic antibodies to human liver endonexin II

### Enzyme linked immunosorbent analysis (ELISA) of rabbit antiserum

To investigate whether immunization of rabbits with purified recombinant human liver endonexin II could lead to development of anti-idiotypic antibodies, reacting to its specific receptor ligand, rabbits immunized with native or recombinant human liver endonexin-II (see Materials and Methods) were bled at intervals and examined by ELISA to determine the possible presence of anti-HBs activity. As shown in figure 17, anti-HBs and anti-endonexin II activity was demonstrated in serum. All four rabbits (two immunized with native human liver endonexin-II, two immunized with recombinant human liver endonexin-II) showed comparable anti-HBs activity. This activity was specific, since pre-immune serum did not react with major HBsAg and anti-HBs activity (as determined by ELISA) could be eliminated by preincubation of the antiserum with excess major HBsAg (figure 18). In contrast, rabbits, immunized with the same amounts of rat liver endonexin-II, which does not have HBsAg-binding properties despite 90% sequence homology as compared to human liver endonexin-II did not develop anti-HBs antibodies, but did develop a comparable antie-E-II response at the same moment (Figure 17, Panel B).

### Separation of idiotypic and anti-idiotypic antibodies.

To exclude the possibility of crossreactivity of anti-endonexin-II to HBsAg epitopes, the anti-HBs and anti-endonexin-II activities, present in rabbit serum, were separated by affinity chromatography using recombinant endonexin-II coupled to CNBr-activated Sepharose (see Materials and Methods). Flow-through fractions (1-12) as well as eluted fractions (13-18) from the affinity column were collected and examined for their reactivity with major HBsAg and endonexin-II (ELISA). As demonstrated in figure 19, the anti-HBs activity was predominantly found in the flow-through fractions (1-6), while anti-endonexin-II reactivity was exclusively found in the eluted fractions (14-17), indicating that the anti-HBs reactivity in rabbit anti-endonexin-II antibodies is directed to major HBsAg epitopes.

### Immuno dot-blot analysis.

To verify the above mentioned results as determined by ELISA, major HBsAg and endonexin-II were spotted on nitrocellulose filters and incubated with different antisera. Figure 20 shows that rabbit anti-serum is reactive to both human liver E-II and major HBsAg, while the flow-through fraction of protein A and endonexin-II column chromatography (= purified anti-HBs antibodies) is only reactive to major HBsAg.

### Immunoprecipitation of radiolabeled major HBsAg.

To investigate whether antibodies, separated as above mentioned, were also reactive to antigens in solution, experiments were performed using radiolabeled human liver endonexin-II and major HBsAg respectively. As shown in figure 21, both radiolabeled proteins could be precipitated by serum of rabbits immunized with human liver endonexin-II, while control experiments with pre-immune sera showed absence of precipitation of these radiolabeled proteins. Radiolabeled major HBsAg was incubated in a 1:100 dilution of several anti-HBs antibodies (or control sera) and allowed to stand overnight at 4°C. Subsequently, the mixture was incubated with protein A-Sepharose (Pharmacia) for 3 hours at 4°C. After intensive washing, protein A-Sepharose was pelleted and transferred to counting tubes. The precipitation of labeled proteins was reduced by the addition of unlabeled excess of respective proteins.

### Competitive binding experiments.

To examine whether the generated anti-HBs antibody is an anti-idiotypic antibody (Ab2) that reacts with a specific epitope(s) on major HBsAg that binds to human liver endonexin-II, competitive binding studies were performed using Ab2 immobilized on high bond ELISA dishes. For these experiments, high bond ELISA dishes were coated with 500ng of Protein A and endonexin-II column chromatography-purified anti-idiotypic antibodies, without anti-endonexin-II reactivity (see Figure 15), followed by incubation with PBS/Gelatin 3% (Merck, Darmstadt, Germany) to block non-specific binding sites and washing with PBS/Tween 20-0.05%. Wells were then incubated with binding mixtures for 1 hour at room temperature. After incubation, wells were washed intensively with PBS/Tween 20-0.05%, Bound radiolabeled HBsAg was released in SDS-PAGE sample buffer and transferred to counting tubes.

As shown in figure 22, human liver endonexin-II succesfully competes with the anti-HBs antibody for binding to major HBsAg in the presence of Ca²⁺ and Mg²⁺ . It is therefore concluded that the anti-HBs antibody mimics a region of endonexin-II that interacts with major HBsAg.

### EXAMPLE 11 : Peptides derived from endonexin II showing HBsAg binding properties

### Limited acid hydrolysis of endonexin II

Purified endonexin-II was treated according to the method as described in example 3. (Hydrolysis in 3% formic acid at 112°C for 4h). After treatment with formic acid, the protein digest was separated by reversed phase HPLC on a Vydac C-4 column (2.1 x 250 mm, Hesperia, CA, USA) as described in example 3. Peptide peaks were registered on a chart recorder and collected manually. Multiple digestions and separations were performed and elution patterns (comparable to that shown in figure 3) were carefully compared to identify identical peak fractions for binding experiments and sequencing.

### Binding of radiolabeled human endonexin II to recombinant major HBsAg (rHBsAg) in the presence of endonexin-II peptide fractions.

Binding experiments were performed according to the method as described in example 1 and 5. However, binding of radiolabeled endonexin-II to immobilized major HBsAg was carried out in the presence of individual peak fractions of unlabeled digested human liver endonexin-II (see above). The experiment was performed in triplicate. Based on these results, four peptide fraction (peaks F, Q, Q, R, S) were found to inhibit binding of radiolabeled endonexin-II to major HBsAg significantly more than the others (figure 23A). Therefore, these peptide was considered as one of the important major HBsAg-binding epitopes on endonexin-II. By sequencing an identical peak fraction in a parallel experiment, peptides corresponding to peaks F and S were determined as amino acids 266 to 280 (peak F, DHTLIRVMVSRSEID) and amino acids 92-138 (peak S): DAYELKHALKGAGTNEKVLTEIIASRTPEELRAIKQVYEEEYGSSLE) of human endonexin II (numbering according to Kaplan et al., 1988). Also peaks L and N, showing a slightly lower binding inhibition capacity were analysed and found to correspond to the region encompassing amino acids 190 to 225 of human endonexin (DEEKFITIFGTRSVSHLRKVFDKYMTISGFQIEETI).

Subsequently, a synthetic peptide based on the sequence of peptide F TLIRVMVSSEIDLF was used to immunize rabbits. Rabbits immunized with this synthetic F-peptide were found to generate a strong anti-peptide response. Intact endonexin II was not reactive with these antibodies. However, a high titer of anti HBs anti bodies (Ab2=anti-idiotypic antibodies) was again found in the rabbit antiserum, suggesting that an epitope(s) within this peptide can indeed be involved in major HBsAg binding.

### Isolation of F(Ab')2 fragment of anti-endonexin-II antibodies.

After affinity purification of antibodies using endonexin-II and subsequent Protein A column chromatography, the IgG preparation was digested with pepsine according to standard protocols (Gorevic et al., 1985). After digestion, the preparation was fractionated by Protein A column chromatography. Flow-through fractions, containing F(Ab')2 fragment, were used to immunize chickens.

### Immunization of chickens with F(Ab')2 fragment.

Chickens were immunized according to standard immunisation protocols. Pre-immune IgY were isolated from eggs (yolk) prior to the first injection. Eggs were collected and used for isolation of IgY as described by Jensenius et al. (1981) Screening of generated antibodies was performed by ELISA as described above. Results of these ELISA (figure 23B) clearly demonstrate the generation of anti-HBs antibodies (anti-idiotypic), after injection of anti-endonexin-II F(Ab')2 fragments (idiotypic). This finding further supports the concept that endonexin-II and major HBsAg have a unique receptor-ligand relationship.

### Competitive binding experiments between anti-idiotypic antibodies.

To investigate wether the anti-HBs antibodies, generated by intact human liver endonexin-II (Ab2/E-II), synthetic F peptide (Ab2/F) or F(Ab')2 fragment (Ab2/FAb), recognize identical epitopes on major HBsAg, a competitive binding assay was performed (see figure 23C). For this purpose, affinity purified Ab2/E-II (E-II refers to human liver endonexin II) was radiolabelled and added to major HBsAg, immobilized on high bond ELISA dishes, in the presence or absence of unlabelled Ab2/E-II, Ab2/F, Ab2/FAb, irrelevant IgG as control protein or major HBsAg. As shown in figure 23D binding of radiolabeled Ab2/E-II can be inhibited by addition of unlabeled major HBsAg or unlabeled Ab2/E-II, Ab2/F, Ab2/FAb in a dose dependent manner, while rabbit IgG did not inhibit the binding of radiolabeled Ab2/E-II to major HBsAg. Therefore, it can be concluded that anti-HBs antibodies, generated by immunisation of rabbits with intact human liver endonexin-II or synthetic F-peptide and by chickens immunized with anti-E-II F(Ab')2 fragment, recognize identical regions on major HBsAg. Since previous results also demonstrate that Ab2/E-II competes with human liver endonexin-II for binding to major HBsAg, it can be concluded that the F-peptide is involved in binding of major HBsAg to human liver endonexin-II.

### LIST OF REFERENCES

Bianchi R, Giambanco I, Ceccarelli P, Pula G, Donato R (1992) Membrane-bound annexin V isoforms (CaBP33 and CaBP37) and annexin VI in bovine tissues behave like integral membrane proteins. FEBS Letters 296:158-162.
Blalock J (1990) Complementarity of peptides specified by 'sense' and 'antisense' strands of DNA. Trends Biotechnol 8:140-144.
Bradford MM (1976) A rapid and sensitive method for the quantitation of microgram quantities of protein utilizing the principle of protein-dye binding. Anal Biochem 72:248-254.
Brummelhuis W, Wilson-de Stürler L, Raap A (1981) In: Hepatitis B vaccine (eds. Maupas P & Amsterdam) p. 51-56 (North Holland Biomedical Press, Amsterdam)
Comera C, Rothhut B, Cavadore JC, et al. (1989) Further characterization of four lipocortins from human peripheral blood mononuclear cells. J Cell Biochem 40:361-370.
Crompton M, Moss S, Crumpton M (1988) Diversity in the lipocortin/calpactin family. Cell 55:1-3.
Dash S, Rao K, Joshi B, Nayak NC, Panda SK (1991) Significance of natural polymerized albumin and its receptor in hepatitis B infection of hepatocytes. Hepatology 13:134-142.
de Préval C (1978) Immunoglobulins. In: Bach J. Immunology. New York, Wiley and Sons: 144-219.
Duchosal A, Eming S, Fischer P et al. (1992) Immunization of hu-PBL-SCID mice and the rescue of human monoclonal Fab fragments through combinatorial libraries. Nature 355:258-262.
Fleischmann J, Davie J (1984) Immunoglobulins: allotypes and idiotypes. In: Paul W.(ed.) Fundamental Immunology. New York, Raven Press:205-220.
Funakoshi T, Hendrickson L, McMullen B, Fujikawa K (1987) Primary structure of human placental anticoagulant protein. Biochemistry 26:8087-8092.
Ganem D (1982) Persistent infection of humans with Hepatitis B virus Mechanisms and consequences. Rev Infect Dis 4:1026-1047.
Ganem D, Varmus HE (1987) The molecular biology of hepatitis B viruses. Ann Rev Biochem 56:651-693.
Gheuens J, MacFarlin D (1982) Use of monoclonal anti-idiotypic antibody to P3-X63Ag8 myeloma protein for analysis and purification of B lymphocyte hybridoma products. Eur J Immunol 12:701-703.
Ghiso J, Saball E, Leoni J, Rostagno A, Frangion (1990) Binding of cystatin C to C4: the importance of antisense peptides in their interaction. Proc Natl Acad Sci USA 87:1288-1291.
Gorevic P, Prelli F, Frangioni B (1985) Immunoglobulin G. Methods Enz. 116: 3-25.
Grundmann U, Abel K, Bohn H, Löbermann H, Lottspeich F, Küpper H (1988) Characterization of cDNA encoding human placental anticoagulant protein (PP4) homology with the lipocortin family. Proc Natl Acad Sci USA 85:3708-3712.
Haigler H, Fitch J, Jones J, Schlaepfer D (1989) Two lipocortin-like proteins, human endonexin II and anchorin CII, may be alternate splices of the same gene. TIBS 14:48-50.
Haigler H, Schlaepfer D, Burgess W, Haigler H (1987) Characterization of lipocortin I and an immunologically related 33-kD protein as epidermal growth factor receptor (human substrate and phospholipase A2 inhibitors). J Biol Chem 262:6921-6930.
Heermann K, Goldmann W, Schwartz T, Seyffrath H, Baumgarten W, Gerlich W (1984) Large surface proteins of hepatitis B virus containing the pre-S sequence. J Virol 52:396-402.
Hilleman M, McAleer W, Buynack E, McLean A (1983) The preparation and safety of hepatitis B vaccine. J Infect 7 (Suppl.1):3-8.
Hjelmeland L (1980) A nondenaturing zwitterionic detergent for membrane biochemistry: design and synthesis. Proc Natl Acad Sci USA 77:6368-6370.
Hubbard A, Wall D, Ma A (1983) Isolation of rat hepatocyte plasma membranes. J Cell Biol 96:217-229.
Iwarson S, Tabor E, Thomas HC, et al. (1985) Neutralisation of hepatitis B virus infectivity by a murine monoclonal antibody. An experimental study in the chimpanzee. J Med Virol 16:89-96.
Iwasaki A, Suda M, Nakao H, et al. (1987) Structure and expression of cDNA for an inhibitor of blood coagulation isolated from human placenta: a new lipocortin-like proteins. J Biochem 102:1261-1273.
Jensenius J, Andersen I, Hau J, Crone M, Koch C (1981) Eggs: conveniently packaged antibodies. Methods for purification of yolk IgG. J Immunol Meth 46:63-68.
Kaiser J, Stockert J, Schein P, Wolkoff A (1986) Recognition of Hepatitis B surface antigen by the human hepatoma cell line HepG2. Trans Assoc Am Physicians 99:86-91.
Kaplan R, Jaye M, Burgess W, Schlaepfer D, Haigler H (1988) Cloning and expression of cDNA for human endonexin II, a Ca²⁺ and phopholipid binding protein. J Biol Chem 263:8037-8043.
Karshikov A, Berendes R, Burger A, Cavalié A, Lux H, Huber R (1992) Annexin V membrane interaction: an electrostatic potential study. Eur Biophys Journal 20:337-344.
Klebe R, Harries J, Sharp Z (1983) A general method for polyethylene-glycol induced genetic transformation of bacteria and yeast. Gene 25:333-341.
Klee B (1988) Ca²⁺-dependent Phospholipid-and membrane-binding proteins. Biochemistry 27:6645-6653.
Leenders WPJ, Glansbeek HL, De Bruin WCC, Yap SH (1990) Binding of the major and large HBsAg to human hepatocytes and liver plasma membranes: putative external and internal receptors for infection and secretion of hepatitis B virus. Hepatology 12:141-147.
Leenders W, Hertogs K, Moshage H, Yap SH (1992) Host and tissue tropism of hepatitis B virus. Liver 12:51-55.
McAleer W, Buynack R, Maigetter D, Wampler D, Miller W, Hilleman M (1984) Human hepatitis B vaccine from recombinant yeast. J Am Med Assoc 251:2812-2815.
Machida A, Kishimoto S, Ohnuma et al. (1984) A polypeptide containing 55 amino acid residues coted by the pre-S region of hepatitis B virus deoxyribonucleic acid bears the receptor for polymerized human as well as chimpanzee albumins. Gastroenterology 86:910-918.
Mead D, Szczesna-Skoupra, Kemper B (1986) Single-stranded DNA 'blue' T7 promoter plasmids: a versatile tandem promoter system for cloning and protein engineering. Protein engineering Vol. 1, No. 1, p. 67-74.
Meheus L, Van Beumen J, Coomans A, Van Fleteren J (1987) Age specific nuclear proteins in the nematode worm Caenorhabditis elegans. Biochem J 245:257-261.
Moshage H, Rijntjes P, Hafkenscheid J, Roelofs H, Yap S (1988) Primary culture of cryopreserved adult human hepatocytes on homologous extracellular matrix and the influence of monocytic products on albumin synthesis. J Hepatology 7:34-44.
Neurath AR, Kent SBH, Strick N, Parker K (1986) Identification and chemical synthesis of a host cell receptor binding site on hepatitis B virus. Cell 46:429-436.
Neurath AR, Strick N, Sproul P, Ralph HE, Valinsky J (1990) Detection of receptors for hepatitis B virus on cells of extrahepatic origin. Virology 176:448-457.
O'Farrel PZ, Goodman HM, O'Farrel PH (1977) High resolution two-dimensional electrophoresis of basic as well as acidic proteins. Cell 12:1133-1142.
Peeples ME, Komai K, Radek R, Bankowski MJ (1987) A cultured cell receptor for the small S protein of hepatitis B virus. Virology 160:135-142.
Pepinsky R, Tizard R, Mattaliano J, et al. (1988) Five distinct calcium and phospholipid binding proteins share homology with Lipocortin I. J Biol Chem 263:10799-10811.
Petit MA, Dubanchet S, Capel F, Voet P, Dauguet Ch, Hauser P (1991) HepG2 cell binding activities of different Hepatitis B Virus isolates: inhibitory effect of anti-Hbs and anti-preS1(21-47). Virology 180:483-491.
Pfaffle M, Ruggiero F, Hofmann H, et al. (1988) Biosynthesis, secretion and extracellular localization of anchorin CII, a collagen-binding protein of the calpactin family. EMBO J 7:2335-2342.
Pontisso P, Petit MA, Bankowski MJ, Peeples ME (1989a) Human liver plasma membranes contain receptors for the hepatitis B virus pre-S1 region and, via polymerised human serum albumin, for the pre-S2 region. Virol 63:1981-1988.
Pontisso P, Ruvoletto MG, Gerlich WH, Heermann KH, Bardini R, Alberti A (1989b) Identification of an attachment site for human liver plasma membranes on hepatitis B virus. Virology 173:522-530.
Rasmussen HH, Van Damme J, Bauw G, et al. (1991) Protein-electroblotting and microsequencing in establishing integrated human protein databases. Methods in Protein Sequence Analysis. Ed. H. Jörnvall, J. Höög, A. Gustavsson. Advances in Life Sciences p.103-114.
Rijntjes P, Moshage H, Van Gemert P, De Waal R, Yap S (1986) cryopreservation of adult human hepatocytes. The influence of deep freezing storage on the viability, cell seeding, survival, fine structures and albumin synthesis in primary cultures. J Hepatology 3:7-18.
Rink (1987), Solid phase synthesis of protected peptide fragments usinf a trialkoxy-diphenyl-methylester resin. Tetrahedron Letters 28: 3787-3790.
Robinson W, Miller R, Marion P (1987) Hepadnaviruses and retroviruses share genome homology and features of replication. Hepatology 7:64S-73S.
Rojas E, Pollard H, Haigler H, Parra C, Burns L (1990) Calcium-activated human endonexin II forms calcium channels across acidic bilayer membranes. J Biol Chem 265:21207-21215.
Roubos E (1990) Sense-antisense complementarity of hormone-receptor interaction sites. Trends Biotechnol 8:279-281.
Salacinski Ph RP, McLean Ch, Sykes JEC, Clement Jones VV, Lowry Ph J (1981) Iodination of proteins, glycoproteins, and peptides using a solid-phase oxidizing agent, 1, 3, 4, 6-tetra chloro-3a, 6a-diphenyl glycoluril. (Iodogen) Anal Biochem 117:136-146.
Schägger H, von Jagow G (1987) Tricine-sodium dodecyl sulfate-polyacrylamide gel electrophoresis for the separation of proteins in the range from 1 to 100kDa Anal Biochem 166:368-379.
Schlaepfer D, Mehlman T, Burgess W, Haigler H (1987) Structural and functional characterization of human endonexin II, a calcium- and phospholipid-binding protein. Proc Natl Acad Sci USA 84:6078-6082.
Schröder CH, Zentgraf H (1990) Hepatitis B virus related hepatocellular carcinoma chronicity of infection the opening to different pathways of malignant transformation. Biochim Biophys Acta 1032:137-156.
Summers J (1981) Three recently described animal virus models for human hepatitis B virus. Hepatology 1:179-183.
Theilmann L, Goesser T (1991) Interactions of hepatitis B virus with hepatocytes: mechanisms and clinical relevance. Hepatogastroenterol 38:10-13.
Vanfleteren JR, Raymackers JG, Van Bun SM, Meheus LA (1992) Peptide mapping and microsequencing of proteins separated by SDS-PAGE after in situ limited acid hydrolysis. Biotechniques 12:550-557.
Walker JH, Boustead C, Brown R, Koster J, Middleton C (1990) Tissue and subcellular distribution of endonexin, a calcium-dependent phospholipid-binding protein. Biochem Soc Trans 18:1235-1236.
Waters J, Pignatelli M, Galpin S, Ishihara K, Thomas HC (1986) Virus neutralizing antibodies to hepatitis B virus. The nature of an immunogenic epitope on the S gene peptide. J Gen Virol 67:2467-2473.
Waters JA, O'Rourke SM, Richardson SC, Papaevangelou G, Thomas HC (1987) Qualitative analysis of the humoral immune response to the "a" determinant of HBs antigen after inoculation with plasma-derived or recombinant vaccine. J Med Virol 21:155-160.
Zaks W, Creutz C (1990) Evaluation of the annexins as potential mediators of membrane fusion in exocytosis. J Bioener Biomembr 22:97-119.

## Claims

1. Mammalian nonhuman transgenic animal liable to be infected by HBV and moreover liable to suffer from hepatocellular damage, caused by the constitutive or inducible expression of the human endonexin II gene or by the nucleotide sequence coding for its muteins or fragments, the said gene or the said nucleotide sequence being introduced into such a transgenic animal as a model for testing potential drugs which are able
- to modulate the expression of HBV and/or,
- to modulate the binding of HBV to endonexin II and/or,
- to modulate the binding of HBV to a mutein derived from human endonexin II, with said mutein being such that it is liable to bind to large and/or major HbsAg and/or,
- to modulate the binding of HBV to a fragment of human endonexin II with said fragment being such that it is liable to bind to large and/or major HBsAg;
* or with said fragment being such that it is liable to be recognized by antibodies raised against human endonexin II, and capable of neutralizing the HBV infection,
* or with said fragment being such that it is liable to generate antibodies liable to recognize the above-said human endonexin II, or to recognize a variant polypeptide which corresponds to the above-defined receptor, comprising localized mutations such that the variant polypeptide does not lose its property of being a receptor of large and/or major HBsAg and/or,
- to modulate the binding of HBV to endonexin II peptides:
DHTLIRVMVSRSEID,
DAYELKHALKGAGTNEKVLTEIIASRTPEELRAIKQVYEEEYGSSLE, and
DEEKFITIFGTRSVSHLRKVFDKYMTISGFQIEETI
